# EUROPEAN PATENT SPECIFICATION

(11) **EP 0 747 448 B1**
(45) Date of publication and mention of the grant of the patent: **06.11.2002**
(21) Application number: 96303881.5
(22) Date of filing: 30.05.1996
(51) Int. Cl.: C09B 23/04, G01N 33/533, C12Q 1/68

(54) **Rigidized monomethine cyanine dyes**
Versteifte Monomethin-Cyaninfarbstoffe
Colorants cyanines monométhiniques rigidifiés

(30) Priority: 07.06.1995 US 474056; 07.06.1995 US 474057
(43) Date of publication of application: 11.12.1996
(73) Proprietor: CARNEGIE MELLON UNIVERSITY, Pittsburgh Pennsylvania 15213 (US)
(72) Inventor: Waggoner, Alan Stewart, Pittsburgh, PA 15208 (US); Karandikar, Bhalchandra Madhav, Tigard, Oregon 97223 (US); Mujumdar, Ratnakar Balvant, Glenshaw, PA 15116 (US)
(74) Representative: Rollins, Anthony John

(56) References cited:
- WO-A-93/09185
- GB-A- 610 064
- US-A- 2 541 400
- US-A- 4 490 463
- JOURNAL OF CRYSTALLOGRAPHIC AND SPECTROSCOPIC RESEARCH, vol. 21, no. 2, April 1991, pages 179-182, XP000612956 T. RAMOS ET. AL.: "Crystal and molecular structure of 6-phenyl-13H-pyrimido [4,3-b:6,1-b] bis-benzothiazolium-12 triiodide"
- ZEITSCHRIFT FÜR PHYSIKALISCHE CHEMIE, vol. 64, 1969, FRANKFURT AM MAIN, pages 97-114, XP000613001 G. SCHEIBE ET. AL. : "Das Franck-Condon-Prinzip und die Lichtabsorption von Merocyaninen"
- PHOTOGRAPHIC ENGINEERING, vol. 19, no. 5, October 1975, MASSACHUSETS,WASHINGTON D.C. US, pages 273-278, XP002020970 D.M. STURMER; W.S. GAUGH: "Spectral Sensitization by Sterically Hindered Chromophores"

## Description

The present invention relates to chemical dyes which can be used as fluorescent markers. In particular the invention relates to cyanine dye-based compounds which have been rigidized by the inclusion of a bridging group between the heterocyclic rings of the compounds and to methods for their preparation. The subject dyes may be produced or chemically modified to include reactive or other groups, to allow the compounds to covalently or non-covalently associate with a material to thereby impart fluorescent properties to that material.

Fluorescent dyes are generally known and used for fluorescence labelling and detection of various biological and non-biological materials by procedures such as fluorescence microscopy, fluorescence immunoassay and flow cytometry. A typical method for labelling such materials with fluorescent dyes is to create a fluorescent complex by means of bonding between suitable groups on the dye molecule and compatible groups on the material to be labelled. In this way, materials such as cells, tissues, amino acids, proteins, antibodies, drugs, hormones, nucleotides, nucleic acids, lipids and polysaccharides and the like may be chemically labelled and detected or quantitated, or may be used as fluorescent probes which can bind specifically to target materials and detected by fluorescence detection methods.

Four commonly used classes of fluorescent dyes are those based on the fluorescein (green fluorescence) and rhodamine (orange fluorescence) , coumarin and pyrene (blue fluorescence) chromophores. Dyes based on fluorescein have a number of disadvantages, including their tendency to photobleach when illuminated by strong excitation sources. The resulting rapid loss of image with time makes detection and quantitation more difficult with these dyes. Fluorescein derivatives also have a pH-sensitive absorption spectrum and fluorescence yield decreases markedly below pH 8. Rhodamine derivatives are difficult labelling reagents to use and are not particularly fluorescent when bound to proteins. Coumarin and pyrene trisulphonates have broad absorption and emission spectra and relatively low extinction coefficients.

Multiple fluorophores each having a different emission spectrum are commonly used in multiplex detection of fluorescently labelled materials in such procedures as flow-cytometry, microscopy, electrophoresis, etc. In order to reduce the overlap of fluorescence signals, it is desirable to use fluorescent dyes with narrow absorption and emission bands. Dyes based on the coumarin chromophore for example, have broad absorption and emission peaks (as well as having relatively low extinction coefficients) and are therefore not as suitable for such applications.

US Patent No.5268486 discloses that cyanine compounds of the formula (1): 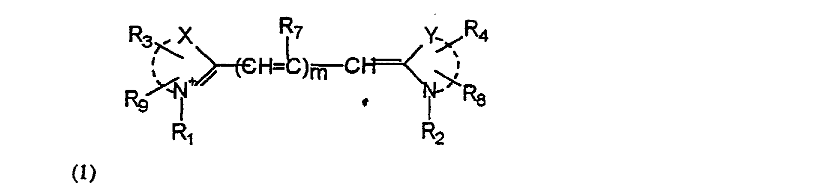 wherein, the dotted lines represent one to three rings having five to six atoms in each ring are useful as fluorescent dyes. R₃, R₄, R₈ and R₉ groups are attached to the rings. At least one of the R₈ and R₉ groups is a sulphonic acid or sulphonate group and at least one of the R₁, R₂, R₃, R₄, and R₇ groups is a moiety that will react with amino, hydroxy, phosphoryl, or sulphydryl groups. These compounds are disclosed as fluorescing in the green, orange, red and near infra-red regions of the spectrum.

"Rigidized" cyanine dyes having a methine group between the heterocycles and based on the bis-pyrromethene boron difluoride structure are described in US Patent Nos.4774339, 5128288, 5248782, 5274113 and 5451663. The basic structure is shown in formula (2): 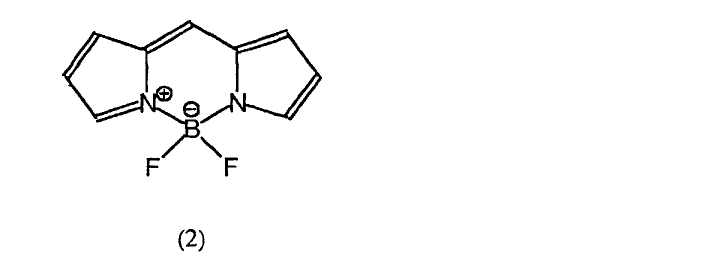 where the pyrrole rings may be substituted. Particular derivatives of this structure include 3,3',5,5'-tetramethyl-2,2'-bispyrromethene-1,1'-boron difluoride, sold under the tradename BODIPY by Molecular Probes Inc, Eugene, Oregon. BODIPY analogues are disclosed in US Patent No.4774339 above. The BODIPY molecules generally fluoresce at wavelengths greater than or equal to 500nm. For example, the '339 patent describes pyrrole-based dyes which absorb and emit with wavelengths comparable with fluorescein, ie, approximately 490 and 500nm respectively.

A pyridine-based monomethine boron difluoride structure of the formula (3) and a quinoline-based monomethine boron complex (formula (4) R= H; Scheibe et al, Z. Phys.Chem., Vol.64, 97-114 (1969)) have also been described. Although the quinoline-based compounds have been evaluated for use as laser dyes, it is unknown whether they are useful as fluorescent markers. Based on its structure, it is believed that the quinoline derivative would have an emission maximum greater than 500nm.

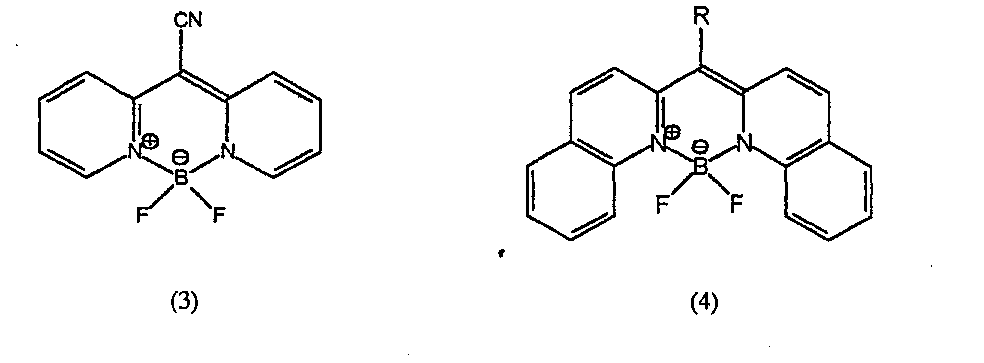

Alkylene-rigidized cyanine dyes of general formula (5) have also been described. See for example: Ramos et al, J.Crystallographic and Spectroscopic Research, Vol.21, No.2, 179-182 (1991); Sturmer and Gaugh, Photographic Science and Engineering, Vol.19, No.5, 273 (1975); British Patent Nos.610064, 618889 (Kodak); US Patent Nos.4490463 (Kodak), 2541400 (Brooker et al) and 3148187 (Heseltine).

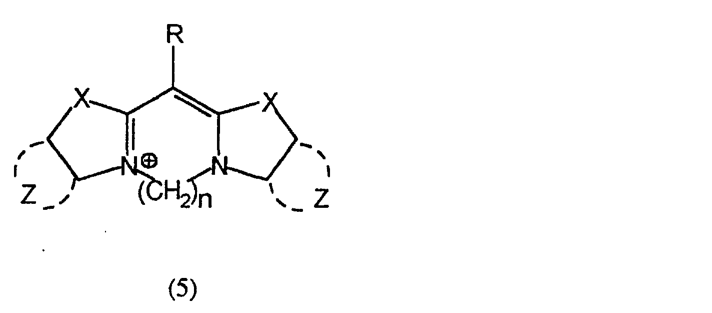

Dyes such as those disclosed above are useful in photographic emulsions as sensitizers. However they cannot be used and have not been described as fluorescent labelling dyes.

None of the foregoing literature discloses fluorescent dye compounds that contain functional groups and/or solubilizing groups which render the dye suitable for covalent labelling, in particular for covalent labelling of biological molecules and other target materials. There is therefore, a lack of bright, soluble fluorescent dye compounds that fluoresce in the shorter wavelength (300-500nm) region of the spectrum, have desirable spectral properties and can be used to label a wide variety of materials.

We have now found a novel class of monomethine, rigidized cyanines that are bright, highly fluorescent, strongly light-absorbing dyes and fluorescent markers that will emit in the near UV and blue (300-500nm) region of the spectrum and that can be used in a variety of biological and non-biological applications.

Accordingly, the present invention relates to rigidized monomethine cyanine fluorescent compounds of the formula (6): 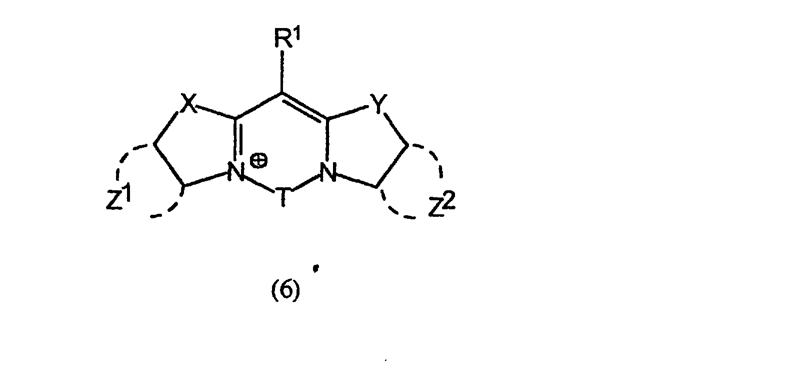 optionally substituted by groups R⁴ to R⁷, or by groups R² to R⁷ when T contains carbon atoms, wherein R² and R³ are attached to the carbon atoms of T and R⁴, R⁵, R⁶ and R⁷ are attached to the rings containing X and Y or, optionally, are attached to atoms of the Z¹ and Z² ring structures;
groups R² to R⁷ which are the same or different are selected from -R⁹ and -L-R⁹ where R⁹ is selected from neutral groups that reduce water solubility, polar groups that increase water solubility, functional groups that can be used in labelling reactions, reactive groups, electron donating and withdrawing groups that shift the absorption and emission wavelengths of the fluorescent molecule, lipid and hydrocarbon solubilising groups, and L is selected from the group consisting of a straight or branched C₁₋₂₆ alkyl chain, a C₂₋₂₀ monoether or polyether and a C₂₋₂₀ atom chain containing up to four secondary amide linkages;
R¹ is selected from hydrogen, aryl, heteroaryl, cyano, nitro, aldehyde, halogen, hydroxy, alkyl groups of twenty-six carbon atoms or less, amino, quaternary amino, acetal, ketal, phosphoryl, sulphydryl, water-solubilizing groups, and -(CH₂)ₙQ where 1<n<26 and Q is selected from amino, substituted amino, quaternary amino, aldehyde, acetal, ketal, halo, cyano, aryl, heteroaryl, hydroxyl, sulphonate, sulphate, carboxylate, amide, nitro, and groups reactive with amino, hydroxyl, aldehyde, phosphoryl, or sulphydryl groups;
T is a linking group such that: 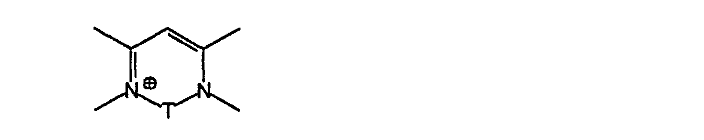 is a six or seven membered ring;
X and Y may be the same or different and are selected from bis-substituted carbon, oxygen, sulphur, selenium, CH=CH, and N-W wherein N is nitrogen and W is selected from hydrogen, a group -(CH₂)ₙR⁸ where n is an integer from 1 to 26 and R⁸ is selected from hydrogen, amino, aldehyde, acetal, ketal, halo, cyano, aryl, heteroaryl, hydroxyl, sulphonate, sulphate, carboxylate, substituted amino, quaternary amino, nitro, primary amide, substituted amide, and groups reactive with amino, hydroxyl, carbonyl, phosphoryl, and sulphydryl groups;
one of groups Z¹ and Z² represents a bond or the atoms necessary to complete one, two fused or three fused aromatic rings each ring having five or six atoms, selected from carbon atoms and, optionally, no more than two oxygen, nitrogen and sulphur atoms and the other group Z¹ or Z² represents the atoms necessary to complete one, two fused or three fused aromatic rings each ring having five or six atoms, selected from carbon atoms and, optionally, no more than two oxygen, nitrogen and sulphur atoms;
provided that at least one of R¹ to R⁷ comprises a reactive group for covalent reaction with a functional group on a target material or comprises a functional group for covalent reaction with a reactive group on a target material.

Suitable neutral groups may be selected from the group consisting of hydrogen and halogen atoms;

Suitable polar groups may be groups selected from the group consisting of amide, sulphonate, sulphate, phosphate, quaternary ammonium, guanidinium, hydroxyl, phosphonate;

Suitable functional groups may be selected from the group consisting of optionally substituted amino, azido, hydroxyl, sulphydryl, imidazole, carboxyl and carbonyl;

Suitable reactive groups may be selected from the group consisting of succinimidyl ester, isothiocyanate, isocyanate, anhydride, haloacetamide, maleimide, sulphonyl halide, phosphoramidite, acid halide, acyl azide, alkylimidate, hydrazide, arylimidate, hydroxylamines, carbodiimides;

Suitable electron donating and withdrawing groups may be selected from the group consisting of amide, cyano, nitro, alkoxy, styryl, aryl and heteroaryl groups;

Suitable lipid and hydrocarbon solubilising groups may be selected from the group consisting of alkyl, aryl and aralkyl groups; and
L is selected from the group consisting of a straight or branched C₁₋₂₆ alkyl chain, a C₂₋₂₀ monoether or polyether and a C₂₋₂₀ atom chain containing up to four secondary amide linkages.

Preferred R⁹ groups are selected from: hydrogen, halogen, amide, C₁-C₆ alkoxy, cyano, aryl, heteroaryl, sulphonate, quaternary ammonium, guanidinium, hydroxyl, phosphonate, optionally substituted amino, azido, sulphydryl, carbonyl, reactive groups, for example, succinimidyl ester, isothiocyanate, anhydride, haloacetamide, maleimide, sulphonyl halide, phosphoramidite, acid halide, alkylimidate, hydrazide and carbodiimide; and groups reactive with amino, hydroxyl, aldehyde, phosphoryl, or sulphydryl groups.

Preferably R¹ is selected from hydrogen, aryl, heteroaryl, cyano, halogen, alkyl groups of twenty-six carbon atoms or less and -(CH₂)ₙQ where 1<n < 26 and Q is selected from amino, aldehyde, hydroxyl and groups reactive with amino, hydroxyl, aldehyde, phosphoryl, or sulphydryl groups.

Bis-substituted carbon includes bis C₁ - C₄ alkyl groups and C₄ - C₅ spiro alkyl groups.

Suitably, T is selected from >CR²R³, -CHR²-CHR³- and BM₂ wherein B is boron and M is fluoro or chloro.

Suitably, X and Y are selected from bis-alkyl substituted carbon including C₄-C₅ spiro alkyl derivatives, oxygen, sulphur, selenium, -CH=CH-, and nitrogen.

Suitably, W is a group -(CH₂)ₙR⁸ where n is an integer from 1 to 6 and R⁸ is selected from hydrogen, amino, sulphonate, carboxylate, aryl, hydroxyl, and groups reactive with amino, hydroxyl, carbonyl, phosphoryl, or sulphydryl groups.

Specific examples of the groups R¹, R², R³, R⁴, R⁵, R⁶ and R⁷ and the groups with which those R-groups will react are provided in Table 1. In the alternative, the R¹, R², R³, R⁴, R⁵, R⁶ and R⁷ may be the functional groups of Table 1 which would react with the reactive groups of a target molecule.

**Table 1 :**

| **Possible Reactive Substituents and Sites Reactive Therewith** | |
|---|---|
| **Reactive Groups** | **Corresponding Functional Groups** |
| succinimidyl esters | primary amino, secondary amino, hydroxyl |
| anhydrides | primary amino, secondary amino, hydroxyl |
| acyl azides | primary amino, secondary amino |
| isothiocyanates, isocyanates | amino, thiols, hydroxyl |
| sulphonyl chlorides, sulphonyl fluorides | amino, hydroxyl |
| substituted hydrazines, substituted hydroxylamines | aldehydes, ketones |
| acid halides | amino, hydroxyl |
| haloacetamides, maleimides | thiols, imidazoles, hydroxyl, amines |
| carbodiimides | carboxyl groups |
| phosphoramidite | hydroxyl |

In addition to those groups listed in Table 1, a number of other groups are possible as reactive substituents in the R¹ - R⁷ positions of the compounds of the present invention. For example, the reactive groups which are especially useful for labelling target components with available amino and hydroxy functional groups include: 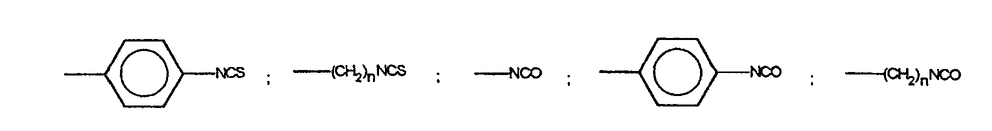 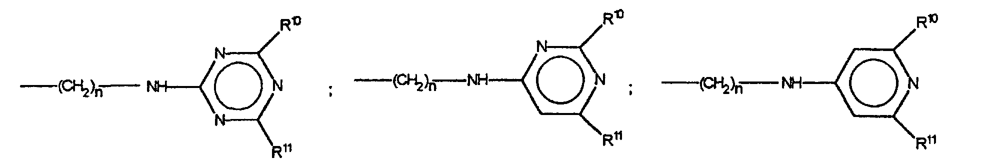 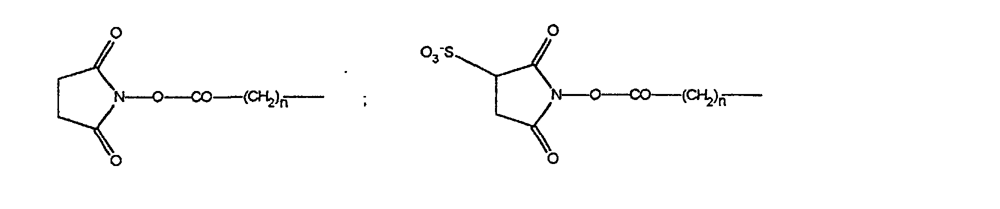 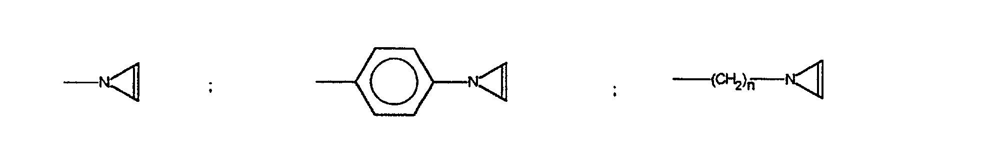 where n = 0 or an integer from 1-10 and at least one of R¹⁰ or R¹¹ is a leaving group such as I, Br, or Cl.

Specific examples of possible R¹, R², R³, R⁴, R⁵, R⁶ and R⁷ groups that are especially useful for labelling target components with available sulphydryl functional groups include: 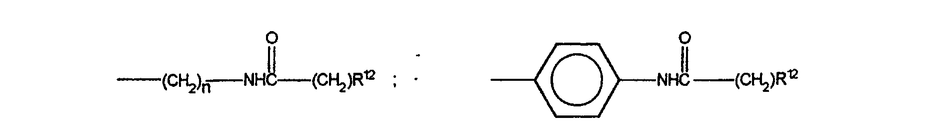 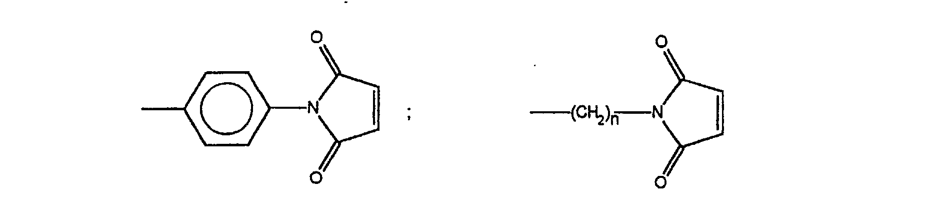 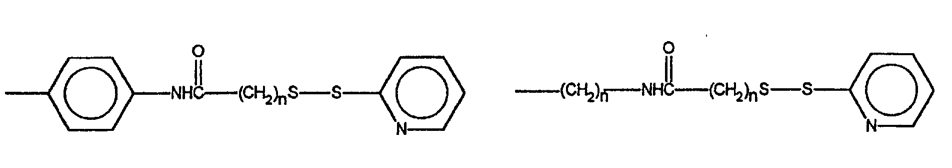 where n=0 or an integer and R¹² is a leaving group such as I or Br.

Specific examples of possible R¹, R², R³, R⁴, R⁵, R⁶ and R⁷ functional groups that are especially useful for labelling target components by light-activated cross linking include:

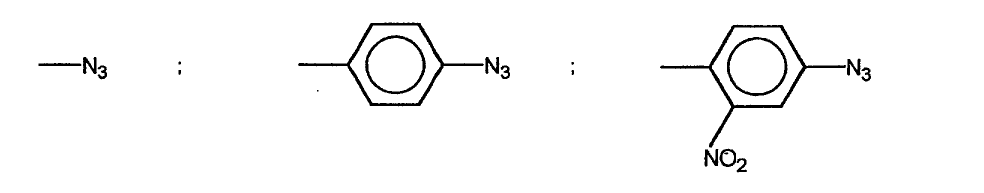

In one preferred embodiment of the present invention the compounds of formula (6) have the formula (7): 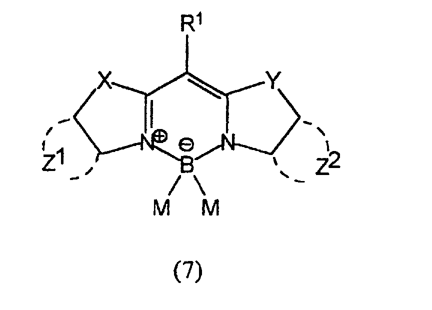 optionally substituted by groups R⁴ to R⁷ wherein R⁴, R⁵, R⁶, and R⁷ are attached to the rings containing X and Y or, optionally, are attached to atoms of the Z¹ and Z² ring structures and wherein R¹, R⁴ to R⁷, X, Y, Z¹, Z² and M are as hereinbefore defined;
provided that at least one of R¹ and R⁴ to R⁷ comprises a reactive group for covalent reaction with a functional group on a target material or comprises a functional group for covalent reaction with a reactive group on a target material.

In a second preferred embodiment of the present invention the compounds of formula (6) have the formula (8): 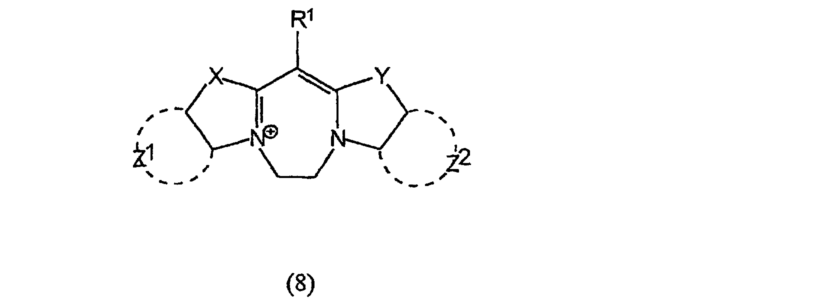 optionally substituted by groups R² to R⁷ where groups R² to R³ are attached to the ethylene linking group, 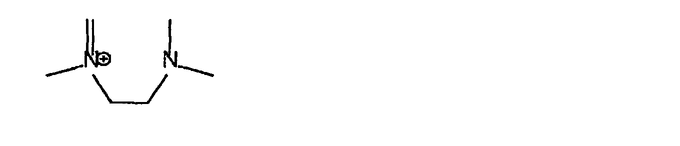 and groups R⁴ to R⁷ are attached to the rings containing X and Y or are optionally attached to atoms of the Z¹ and Z² ring structures, wherein groups R¹, R²-R⁷, X, Y, Z¹ and Z², are as hereinbefore defined;
provided that at least one of R¹ to R⁷ comprises a reactive group for covalent reaction with a functional group on a target material or comprises a functional group for covalent reaction with a reactive group on a target material.

Alkyl is a straight or branched chain alkyl group containing from 1-26 carbon atoms.

Aryl is an aromatic or polyaromatic substituent containing 1-4 aromatic rings having six conjugated carbon atoms and no heteroatoms that are optionally fused to each other or bonded to each other by carbon-carbon single bonds and attached by a single bond and is optionally and independently substituted by straight or branched alkyl chains or polar groups that increase water solubility.

Heteroaryl is a 5- or 6-membered aromatic heterocycle that is optionally fused to additional six-membered rings or is fused to one additional 5- or 6-membered heteroaromatic ring said heteroaromatic rings containing at least 1 and no more than 3 heteroatoms which may be selected from N, O and S, the heteroaryl being attached by a single bond and is optionally and independently substituted by straight or branched alkyl chains or polar groups that increase water solubility.

Aralkyl is a C₁ - C₆ alkyl group substituted by an aryl or heteroaryl group.

Halogen and halo-groups are those selected from chlorine, bromine and iodine.

For the purpose of increasing water solubility or reducing unwanted non-specific binding of the fluorescently-labelled component to inappropriate components in the sample or to reduce interactions between two or more reactive chromophores on the labelled component which might lead to quenching of fluorescence, the R¹, R², R³, R⁴, R⁵, R⁶ and R⁷ functional groups can be selected from the well known polar and electrically charged chemical groups.
Examples of such groups are -E-F- where F is hydroxy, sulphonate, sulphate, carboxylate, substituted amino or quaternary amino, and where E is a spacer group such as -(CH₂)ₙ- where n is 0-6. Useful examples of -E-F groups include C₁₋₆ alkyl sulphonates, such as -(CH₂)₃-SO₃⁻ and -(CH₂)₄-SO₃⁻.

Exemplary compounds of the present invention which demonstrate the capability for adjusting fluorescence colour, water solubility, and the position of the reactive or functional group are as follows:
i) 6,6'-Disulpho-meso-carboxymethyl bis-(benzothiazolyl)methine boron difluoride
ii) α-Carboxymethyl-5,5'-disulpho-3,3'-ethylene-oxacyanine
iii) 3,3'-Ethylene-6-sulphothia-5'-carboxymethyl-6'-sulphooxa monomethine cyanine
iv) 5-Carboxymethyl-bis-(benzoxazolyl)methine boron difluoride
v) α-Carboxymethyl-3,3'-ethylene thiacyanine
vi) *meso*-Carboxymethyl-benzoxazolyl-benzothiazolyl monomethine boron difluoride

The groups provided herein are not meant to be all-inclusive of those groups which can be incorporated at the R sites of the compounds of the present invention. It will be understood that there are various other groups which will react with groups on material that is to be labelled by the compounds of the present invention. Compounds produced by the incorporation of such other groups at the R¹ to R⁷ positions are intended to be encompassed by the present invention.

The compounds of the present invention may be used in numerous biological and non-biological applications. With respect to non-biological applications, compounds of the present invention having one or more uncharged groups at the R¹ to R⁷ positions, for example, C₁₋₂₆ alkyl and aryl moieties may be dissolved in nonpolar materials to provide fluorescent properties to those materials. Such nonpolar materials include, for example, paints, polymers, waxes, oils, inks and hydrocarbon solvents. Another nonbiological application of the present invention is to dissolve compounds of the present invention having one or more charged and/or polar groups at the R¹ to R⁷ positions in polar solvents or other materials such as, for example, water, ethylene glycol, methyl alcohol, or a mixture of water and methyl alcohol. Such charged R-groups include, for example, -NR₃⁺, -SO₃⁻, -PO₃⁻ and -COO⁻, while such polar R-groups include, for example, hydroxyl groups. With respect to biological applications, biological molecules may be noncovalently labelled using the present complexes. For example, complexes of the present invention wherein at least one of R¹ to R⁷ contains a charge, for example, quaternary amino, may be used to noncovalently bind to charged biological molecules such as, for example, DNA and RNA. In addition, compounds of the present invention wherein at least one of R¹ to R⁷ is an uncharged group, for example, a long chain alkyl, may be used to bind to uncharged biological molecules such as, for example, biological lipids.

The dyes of the present invention can also be used as laser dyes according to the procedures set forth in US Patent No.4916711 to Boyer and Morgan. Laser dyes must be fluorescent, must have a quantum yield greater than 0.56 or 0.57 and must be reasonably photostable. The compounds of the present invention satisfy each of these requirements. Further the dyes of the present invention can be used as textile dyes, photographic dyes and as organic conductors.

The complexes of the present invention may also be used to covalently label a target material to impart fluorescent properties to the target material. Covalent labelling using the compounds of the present invention may be utilized either in a biological or a nonbiological application. Examples of target materials that may be labelled in non-biological applications include, for example, cellulose-based materials (including, for example, papers), textiles, petroleum-based products, photographic films, glasses, polymers and gel filtration and chromatography media.

Covalent labelling using compounds of the present invention may be accomplished with a target having at least one functional or reactive group as defined hereinbefore. The target may be incubated with an amount of a compound of the present invention having at least one of R¹ to R⁷ that includes a reactive or functional group as hereinbefore defined that can covalently bind with the functional or reactive group of the target material. The target material and the compound of the present invention are incubated under conditions and for a period of time sufficient to permit the target material to covalently bond to the compound of the present invention.

R¹ to R⁷ can be chosen so that the compounds of the present invention react with different target compounds and/or to have different spectral properties, thereby providing a number of related compounds which can be used in multiplex analyses wherein the presence and quantity of various compounds in a single sample must be differentiated based on the wavelengths and intensities of a number of detected fluorescence emissions. The compounds of the present invention may be made soluble in aqueous, other polar, or nonpolar media containing the material to be labelled by appropriate selection of R-groups.

Complexes of the present invention also have sharp and distinct absorption and emission maxima, a small Stokes shift, and are relatively photostable such that their emission signals do not fade when they are illuminated in a detection system.

The present invention also relates to labelling methods wherein the complexes of the present invention including at least one functional group at the R¹ to R⁷ positions covalently react with amino, hydroxyl, aldehyde, phosphoryl, carboxyl, sulphydryl or other reactive groups of proteins or other materials. Such other materials which can be labelled by the compounds of the present invention include, but are not limited to, nucleic acid, DNA, RNA, blood, cells, microbial materials, and peptides, proteins, drugs, carbohydrates, toxins, particles, plastics or glass surfaces, polymers, and other materials which include amino, hydroxyl, aldehyde, phosphoryl or sulphydryl reactive groups. Widely available automated DNA sequencers, capillary electrophoresis instruments and fluorescence gel readers are examples of instruments for detecting fluorescently labelled materials.

In addition to the foregoing one-step labelling process, the present invention also relates to two-step labelling processes in which, in a first step, a compound of the present invention covalently reacts with and thereby labels a primary component, such as an antibody. In a second or staining step of the two-step procedure, the fluorescently labelled primary component is then used as a probe for a secondary component, such as an antigen for which the antibody is specific. When the target of the so-labelled antibodies is a cell, the second step of the procedure may be used to determine the amount of labelled antibodies which are attached to that type of cell by determining the intensity of the fluorescence of the cells. By this two-step procedure, monoclonal antibodies and other components covalently labelled in the first step with the fluorescent compounds of the present invention could be used as antigen probes.

The compounds of the present invention can also be used to determine the concentration of a particular protein or other component in a system. If the number of reactive groups on a protein which can react with a probe is known, the fluorescence per molecule can be known and the concentration of these molecules in the system can be determined by the total fluorescence intensity of the system. This particular method can be used to measure the concentration of various labelled analytes using microtitre plate readers or other known immunofluorescence detection systems. The concentration of fluorescently labelled material can also be determined using, for example, fluorescence polarization detection instruments.

The fluorescent compounds of the present invention can also be used in a detection method wherein a plurality of the fluorescent compounds are covalently attached to a plurality of different primary components, such as antibodies, each primary component being specific for a different secondary component, such as an antigen, in order to identify each of a plurality of secondary components in a mixture of secondary components. According to this method of use, each of the primary components is separately labelled with a fluorescent compound having a different light absorption and emission wavelength characteristic compared with the dye molecules used for labelling the other primary components. The so-called primary components are then added to the preparation containing secondary components, such as antigens, and the primary components are allowed to attach to the respective secondary components for which they are selective.

Any unreacted probe materials may be removed from the preparation by, for example, washing, to prevent interference with the analysis. The preparation is then subjected to a range of excitation wavelengths including the absorption wavelengths of particular fluorescent compounds. A fluorescence microscope or other fluorescence detection system, such as a flow cytometer or fluorescence spectrophotometer, having filters or monochrometers to select the rays of the excitation wavelength and to select the wavelengths of fluorescence is next employed to determined the intensity of the emission wavelengths corresponding to the fluorescent compounds utilized, the intensity of fluorescence indicating the quantity of the secondary component which has been bound with a particular labelled primary component. Known techniques for conducting multi-parameter fluorescence studies include, for example, multiparameter flow cytometry.

In certain cases a single wavelength of excitation can be used to excite fluorescence from two or more materials in a mixture where each fluoresces at a different wavelength and the quantity of each labelled species can be measured by detecting its individual fluorescence intensity at its respective emission wavelength. If desired, a light absorption method can also be employed.

The detection method of the present invention can be applied to any system in which the creation of a fluorescent primary component is possible. For example, an appropriately reactive fluorescent compound can be conjugated to a DNA or RNA fragment and the resultant conjugate then caused to bind to a complementary target strand of DNA or RNA. Appropriate fluorescence detection equipment can then be employed to detect the presence of bound fluorescent conjugates.
The present invention also relates to the covalent reaction between compounds of the present invention, and amine, hydroxy, aldehyde, sulphydryl, phosphoryl or other known functional groups on materials such as, for example, proteins, peptides, carbohydrates, nucleic acids, derivatized nucleic acids, lipids, certain other biological molecules, biological cells, soluble polymers, polymeric particles, polymer surfaces, polymer membranes, glass surfaces and other particles and surfaces. Because detecting fluorescence involves highly sensitive optical techniques , the presence of these dye "labels" can be detected and quantitated even when the label is present in very low amounts. Thus, the dye labelling reagents can be used to measure the quantity of a material that has been labelled.

Compared with, for example, the fluoresceins, the rigidized monomethine cyanines of the present invention are particularly photostable and are insensitive to pH changes between pH2 and pH10. The compounds of the present invention maximally absorb and emit light at wavelengths between 300 and 500nm or less and are therefore alternatives to coumarins and pyrenes. Also, the approximate 300-500nm emission maxima of the compounds of the present invention correspond to the "blue" region of the visible spectrum and is therefore generally lower than the BODIPY compounds, quinoline-based monomethine cyanine complexes, and pyridine-based monomethine cyanine complexes discussed above, which have absorption and emission maxima of 500nm or greater.

The present invention also provides a process for the preparation of a complex of the formula (6) which comprises the reaction of a compound of formula (A) or a protonated form thereof: 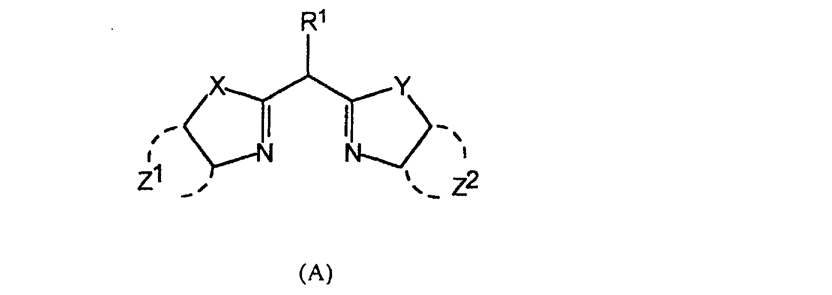 optionally substituted by groups R⁴ to R⁷ wherein R¹ and R⁴ to R⁷, X, Y, Z¹ and Z² are as hereinbefore defined, with a compound suitable for the formation of linkage T, wherein T is as hereinbefore defined.

In a preferred embodiment, the invention provides a process for the preparation of a compound of formula (6) which comprises reaction of a boron compound BM₃ wherein M is fluoro, chloro, bromo or iodo with the quaternised derivative of compound (A), see schemes 1b and 1c. The reaction is suitably carried out in an inert non-polar solvent, for example a hydrocarbon such as toluene. The reaction is suitably carried out in a base, for example an organic base such as diisopropylethylamine at an elevated temperature, for example 50 to 150°C and suitably 100 to 125°C. BM₃ is suitably boron trifluoride etherate.

In a second preferred embodiment, the invention provides a process for the preparation of a compound of formula (7) which comprises reaction of a compound of formula:

R²-CHK-CHK-R³

wherein R² and R³ are as hereinbefore defined and K is a leaving group selected from bromo and para-toluene sulphonate with a compound of formula (A)

Quaternisation of the compounds of formula (A) may be suitably carried out in the presence of an acid HR wherein R is an acid residue, for example halide, ClO₄⁻, CF₃CO₂⁻, or para-toluene sulphonyl unless groups R⁴ to R⁷ are sufficiently electron withdrawing to form a quaternary ammonium ion without the need for the presence of acid. Suitably R is halide eg. bromide. The quaternisation reaction will suitably be carried out at room temperature or up to 250°C.

Symmetric compounds of the formula (A) wherein X and Y are the same and structures Z¹ and Z² are the same may be prepared by a cyclocondensation reaction in which a compound of formula (B), 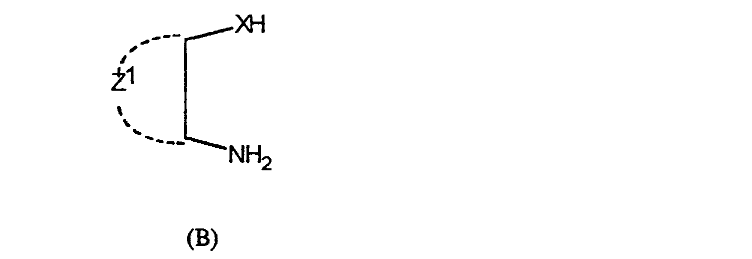 optionally substituted by groups R⁴ and R⁵ wherein R⁴, R⁵, X and Z¹ are as hereinbefore defined, is reacted in appropriate stoichiometry with at least one compound selected from CH₂(CN)₂, CH₂(COOH)₂ and CH₂(COOEt)₂. The reaction is suitably carried out in the presence of polyphorphoric acid and heat. In the alternative, compound (B) may undergo a cyclocondensation reaction with a compound of formula, 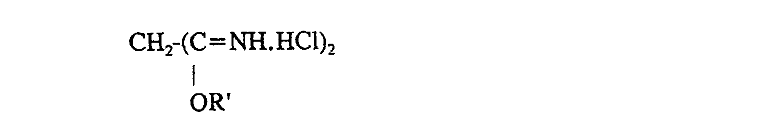 wherein R' is selected from methyl, ethyl, propyl and n-butyl. Suitably the reaction is carried out in the presence of a base such as triethylamine and by heating under reflux in an alcohol such as methanol, see Scheme 1a.

Asymmetric compounds of formula (A) wherein X and Y are different may be prepared by the reaction of a compound of the formula (D): 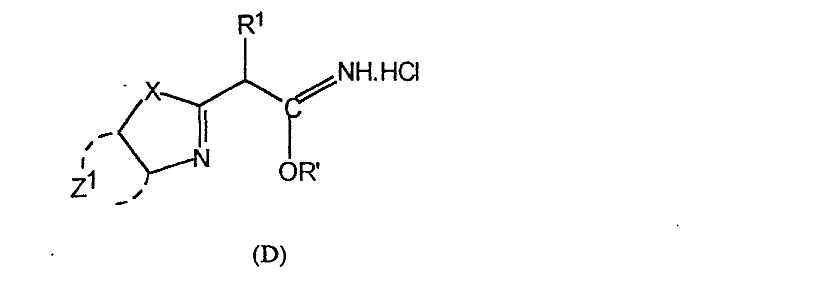 optionally substituted by groups R⁴ and R⁵ wherein R¹, R⁴, R⁵, X and Z¹ are as hereinbefore defined and R' is selected from methyl, ethyl, n-butyl and propyl, with a compound of the formula (E): 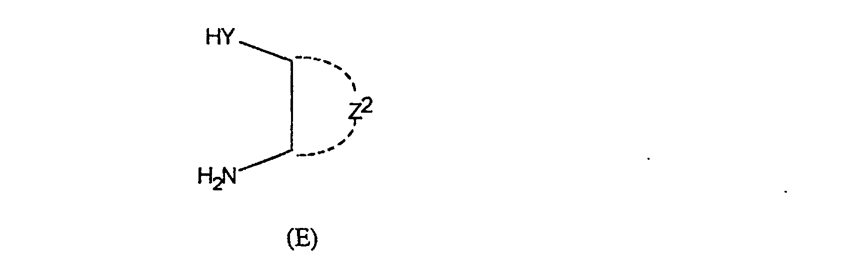 optionally substituted by R⁶ and R⁷ wherein R⁶, R⁷, Y and Z² are as hereinbefore defined, by heating under reflux in solution with an alcohol such as methanol, see Scheme 1a.

The synthetic methods shown in the reaction Scheme la will also be suitable for the preparation of the symmetric compounds of formula (A) by reaction of a compound of the formula (D) with the compound of formula (B) optionally substituted by groups R⁴ and R⁵ wherein R⁴, R⁵, X and Z¹ are as hereinbefore defined.

Precursor compounds of chemical formula (B), (C), (D) and (E) may be prepared by methods well known to those skilled in the area, see for example, US Patent No.4,064,136 to Loew et al.

In order to prepare a compound of formula (5) wherein R¹ is other than hydrogen a compound of formula (A) in which R¹ is hydrogen is reacted in appropriate stoichiometry with a compound GR¹ where G is selected from chlorine, bromine and iodine and R¹ is other than hydrogen in the presence of a base for example NaH, NaOMe, or NaOEt.

It will be readily appreciated that certain compounds of formula (5) may be useful as intermediates for conversion to other compounds of the formula (5) by methods well known to those skilled in the art. Likewise, certain of the intermediates may be useful for the synthesis of derivatives of formula (5). The compounds of the present invention may be synthesized by the methods disclosed herein. Derivatives of the compounds having a particular utility are prepared either by selecting appropriate precursors or by modifying the resultant compounds by known methods to include functional groups at a variety of positions. As examples, the complexes of the present invention may be modified to include certain reactive groups for preparing a fluorescent labelling reagent, or charged or polar groups may be added to enhance the solubility of the compound in polar or nonpolar solvents or materials. As examples of conversions an ester may be converted to a carboxylic acid or may be converted to an amido derivative.

The following are specific examples of the synthesis of compounds of the present invention and observed spectral data for those compounds.

### Example 1 Bis-(Benzothiazolyl)methine boron difluoride

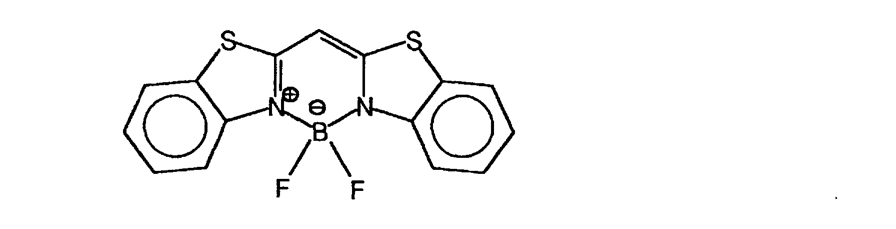

i) In a two-neck 100ml round bottomed flask equipped with a condenser and a stirrer bar, malononitrile (2.64g, 40mmol) was dissolved in absolute ethanol (40ml). 2-Aminothiophenol (10g, 80mmol) was slowly added to this solution under stirring. Under a nitrogen blanket, the reaction mixture was heated under reflux for 6 hours. After cooling, the flask was refrigerated overnight. Pale green crystals of bis-(2-benzothiazolyl)methane that formed were recovered following vacuum filtration, washing with hexane and drying (yield: 82%).
ii) Bis-(2-benzothiazolyl)methane (2.82g, 10mmol) was dissolved in chloroform (50ml) in an Erlenmeyer flask. Hydrobromic acid (10 mmol) in glacial acetic acid was added dropwise with gentle stirring. A canary yellow precipitate formed causing thickening of the reaction mixture. Stirring was continued for one hour at room temperature. A fine yellow powder was recovered following filtration and washing with ether. The yield was quantitative and the bis-(2-benzothiazolyl)methene monohydrobromide product was sufficiently pure for the next stage.
iii) Bis-(2-benzothiazolyl)methine monohydrobromide (1.1g, 3mmol) was suspended in dry toluene (50ml) in a round bottomed flask equipped with a stirrer bar. N,N-Diisopropylethylamine (1.6ml, 9mmol) was added slowly to the suspension under stirring. The suspension became clear and colourless. Using a syringe, boron trifluoride etherate (1.1ml, 9mmol) was added carefully to the clear solution. The reaction mixture turned immediately yellow and some solid separated out. Under a nitrogen atmosphere, the flask was heated on a steam bath for one hour, cooled, and the contents quenched with water (50ml). The toluene layer was separated and stored in a refrigerator to separate a small quantity of yellow solid. The toluene layer was then filtered to remove solid particulates, and the filtrate was evaporated on a rotary evaporator to yield a yellow solid that was redissolved in acetone (20ml). Solid material insoluble in acetone was filtered off and the desired bis-(2-benzothiazolyl)methine boron difluoride complex crystallized from the filtrate (yield: 80%).

### Example 2 Bis-(Benzoxazolyl)methine boron difluoride

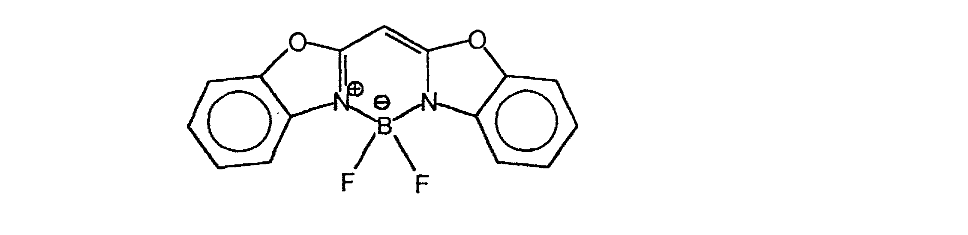

Ortho-aminophenol was condensed with malonic acid in polyphosphoric acid medium by the method of US Patent No.3250780, to produce bis-(benzoxazolyl)methane in a 32% yield. An alternative to malonic acid in the condensation reaction is diethyl malonate.

The condensate was quaternized with hydrobromic acid and was subsequently reacted with boron difluoride as in Example 1 to form the boron rigidized complex.

The observed absorption maxima, molar extinction coefficients, emission spectra and qualitative solubility data for the boron rigidized complexes of Examples 1 and 2 are provided in Table 2.

From Table 2 it is evident that the absorption and emission maxima are practically insensitive over a wide range of solvent polarity. Both compounds have relatively small Stokes' shifts, the oxazole-based compound having a 28nm shift and the thiazole-based compound having only a 5-7nm shift.

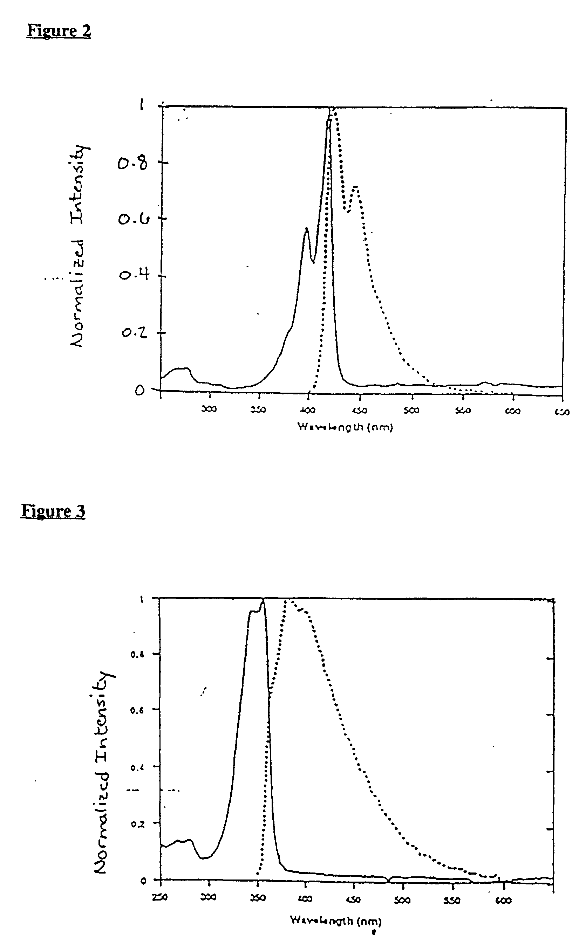

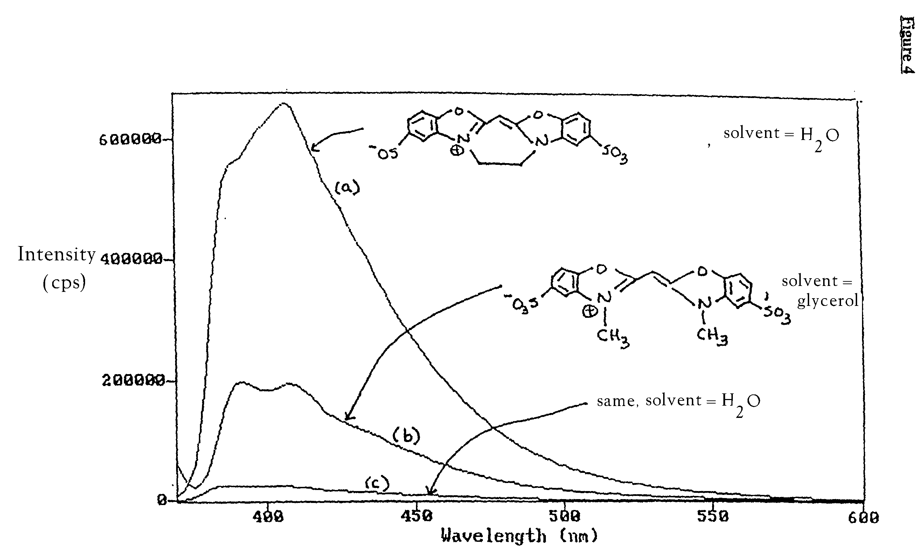

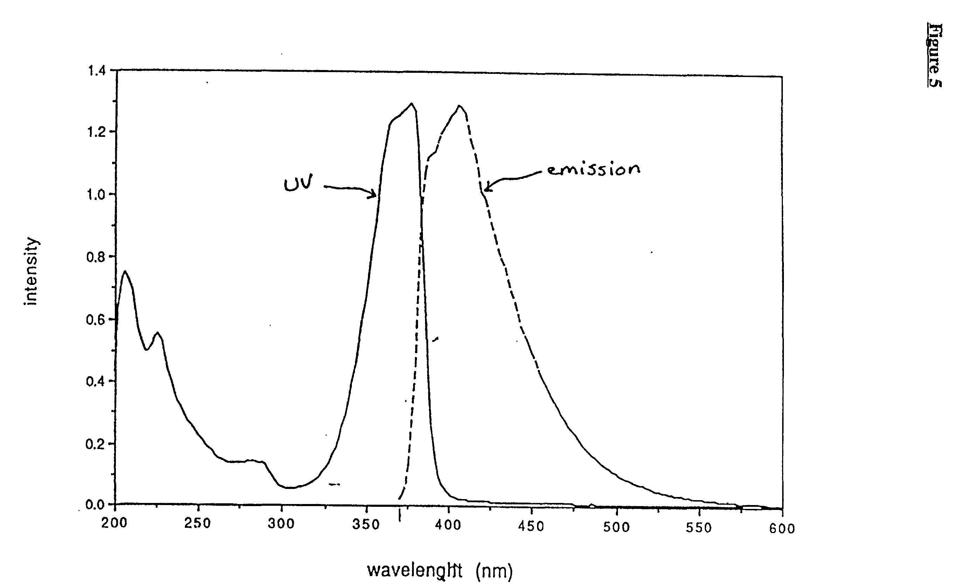

As shown in Fig.2 (thiazole compound of Example 1) and Fig.3 (oxazole compound of Example 2), the absorption spectra (solid line) and emission spectra (dotted line) for the compounds are characterized by sharp and narrow absorption peaks and emission peaks which are somewhat broader.

Relative fluorescence intensities of the compounds of Examples 1 and 2 in a variety of solvents are provided in Table 3. Table 3 indicates that the fluorescence intensities of the dyes are insensitive to solvent polarity. Extremely high fluorescent quantum efficiencies were also observed for both dye compounds.

The resistance to photodegradation of the compounds of Examples 1 and 2 in methanol and dichloromethane was also investigated and was compared with the photofading rate of Coumarin-30. A degassed solution of each dye in a quartz cuvette was illuminated with a 500 watt mercury vapour lamp from a distance of 4 inches. The solution optical density at maximum wavelength was monitored as a function of time. In methanol, the oxazole-based compound and Coumarin-30 faded at a similar rate, while the thiazole-based compound faded somewhat faster. The compounds of Examples 1 and 2 dissolved in dichloromethane, were more resistant to photofading than Coumarin-30.

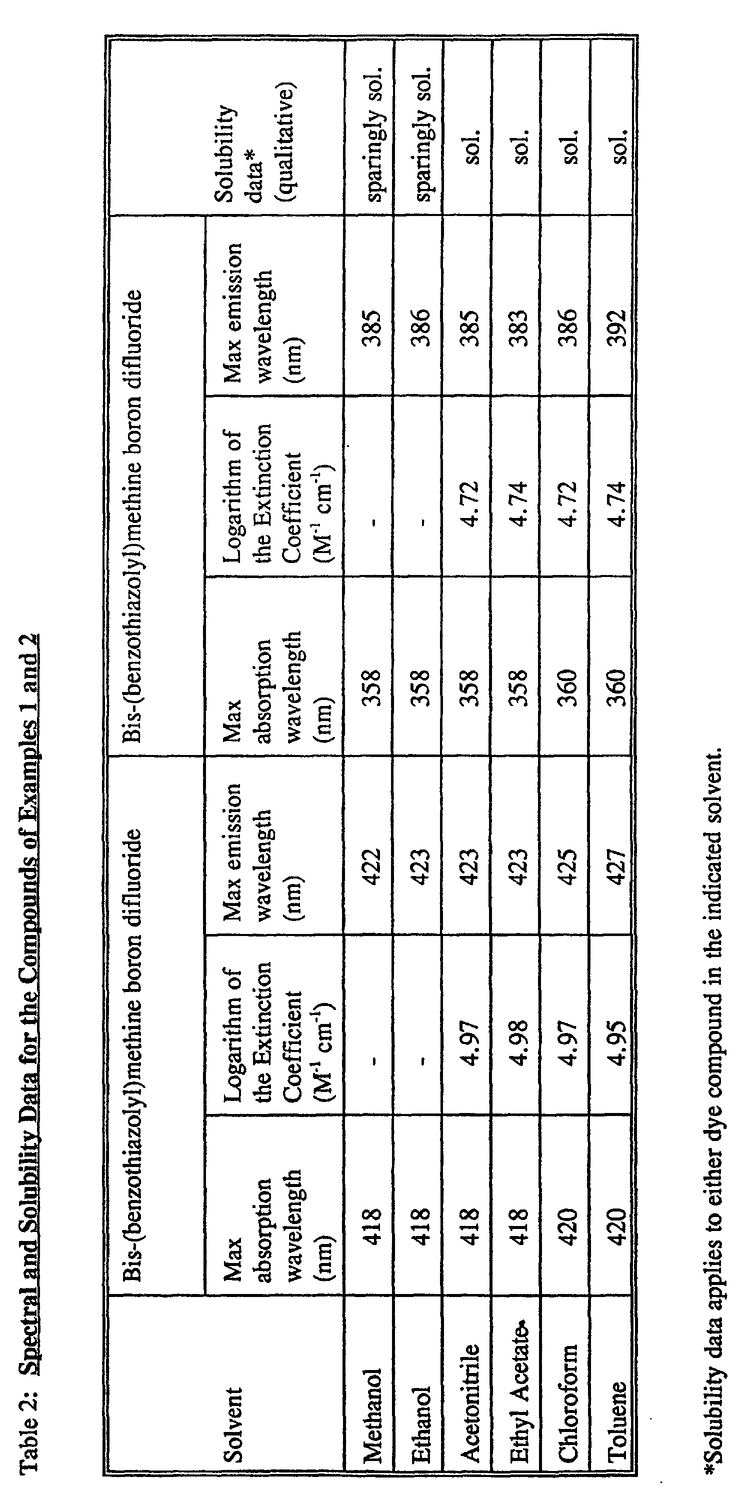

**Table 3:**

| **Relative Fluorescence Intensity Data for the Compounds of Examples 1 and 2** | | |
|---|---|---|
| Solvent | Bis-(benzothiazolyl)methine boron difluoride | Bis-(benzoxazolyl)methine boron difluoride |
| Methanol | 0.89 | 1.35 |
| Ethanol | - | 1.33 |
| Acetonitrile | 0.89 | 1.39 |
| Ethyl Acetate | 0.97 | 1.2 |
| Chloroform | 0.91 | 1.27 |
| Toluene | 0.81 | 1.42 |

### Example 3 Bis-(carboxymethylbenzoxazolyl)methine boron difluoride

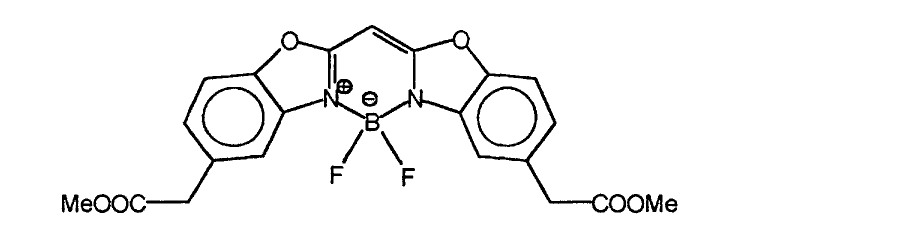

i) To a magnetically stirred solution of 4-hydroxyphenylacetic acid (100g, 0.65mol), in glacial acetic acid (250ml) maintained at 45°C, was added dropwise a mixture of 40ml of nitric acid (specific gravity 1.4) and 60ml of glacial acetic acid. Following addition of the nitric acid/acetic acid mixture, stirring of the resulting mixture was continued for one hour at 25°C and then the flask was chilled in ice water for one hour. The resulting crystals were washed with cold water and air dried at room temperature to give pure 3-nitro-4-hydroxyphenylacetic acid (yield: 65%).
ii) 3-Nitro-4-hydroxyphenylacetic acid (19.7g, 90.1mmol) was dissolved in 160ml of aqueous 0.625M sodium hydroxide solution. Palladium on charcoal 175mg, 10% by weight of the catalyst) was added to the resulting solution and then 2.5 equivalents of hydrazine hydrate was carefully added dropwise using a syringe over 0.5 hours. The temperature of the mixture was observed to increase to 60°C due to the reaction. The reaction mixture was further heated to 80°C and held constant at that temperature for 0.5 hours, followed by refluxing for one additional hour. During the refluxing, the orange colour of the solution was observed to gradually disappear. After reflux, the'reaction vessel was cooled to 25°C and the mixture filtered over Celite to remove the catalyst. The excess solvent was removed from the filtrate to yield 20-30ml of a concentrate, the pH of which was adjusted to 4-5 with glacial acetic acid. On chilling the acidified concentrate, crystals of 3-amino-4-hydroxyphenylacetic acid began to separate out and collected by filtration. A second crop of crystals was recovered on chilling the acidified concentrate overnight in a refrigerator. 3-Amino-4-hydroxyphenylacetic acid was recovered in 92% yield.
iii) Malononitrile (0.66g, 10mmol) was dissolved in 5ml of dry dioxane. To the solution was added 0.92g (20mmol) ethanol, followed by the injection in one portion of 5ml of a 4M solution of hydrochloric acid in dioxane. The reactants were stirred for 36 hours at room temperature. The resulting thick white slurry was filtered, washed with at least three portions of dry ether and dried under vacuum at room temperature for 1-2 hours to give a 93% yield of ethyl bisimidate hydrochloride.
iv) 3-Amino-4-hydroxyphenylacetic acid (1.67g, 10mmol) was suspended in 30ml of dry methanol in a round bottomed flask. 1.15g (5mmol) of the freshly prepared ethyl bisimidate hydrochloride was quickly added to the suspension and the temperature of the resulting mixture was raised to reflux when a second portion of 30ml of dry methanol was added. It was observed that within minutes the solution became clear. As refluxing progressed, the product, bis-(carboxymethylbenzoxazolyl)methane, came out of solution and increased the turbidity of the mixture. Refluxing was maintained for 4 hours, after which time the flask was cooled to room temperature and was then cooled in a refrigerator for 12 hours. Bis-(Carboxymethylbenzoxazolyl)methane was recovered in 75% yield in the form of a white powder following filtration, washing with methanol and drying.
v) The product from the previous step (1.65g, 4mmol) was suspended in methanol (25ml). Acetyl chloride (1ml) was added in one portion and the suspension immediately became clear. The reaction mixture was heated and maintained at reflux for 3 hours, a white solid forming after 0.5 hours. The reaction vessel was cooled to 25°C and then the methanol was removed under vacuum. Ethyl acetate (25ml) was then added to the wet white solid, followed by 20ml of 0.5M aqueous sodium hydroxide. After vigorous mixing of the aqueous and organic layers of the mixture, the organic layer was collected, dried over anhydrous magnesium sulphate and concentrated under a vacuum to provide a colourless viscous oil which included the dimethyl ester of bis-(carboxymethylbenzoxazolyl)methane. This oil was used in the next step.
vi) The hydrobromide quaternary salt of the dimethyl ester of bis-(carboxybenzoxazolyl)methane was prepared by the action of hydrobromic acid. The quaternary salt was then condensed with born trifluoride using a procedure identical to that for the compound of Example 1.
vii) The product of step vi) was suspended in a mixture of 35ml methanol and 5ml of sodium hydroxide (80mg/ml). The suspension was heated under reflux for 0.75 hours and, after cooling, the solvent was partially removed under vacuum to yield 3ml of a concentrate. The pH of the concentrate was adjusted to 4-5 with glacial acetic acid to precipitate out the product. Bis-(carboxymethylbenzoxazolyl)methine boron difluoride was recovered in the form of a white powder following filtration of the concentrate, washing with cold water and drying in a vacuum at 25°C (yield: 88%). ¹H NMR in CDCl₃: δ, 7.6 (m, 4H, 4-H, 6-H, 4'-H, 6'-H); 7.3 (d, 2H, J=7Hz, 7-H, 7'-H); 6.2 (s, 1H, methine H); 3.8 (s, 4H, 2 CH₂-COOH).

The maximum observed absorptive wavelength for the compound was 362nm and the maximum emissive wavelength was 386nm, both measured in methanol. The compound was highly fluorescent.

### Example 4 Benzoxazolyl-benzothiazolyl-methine boron difluoride

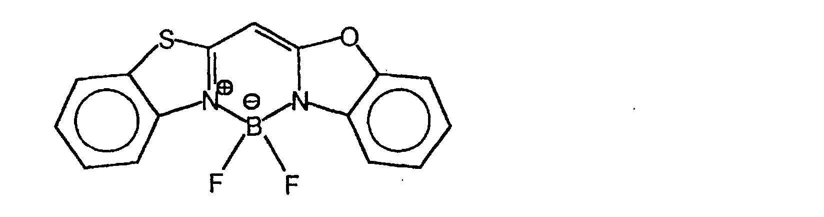

i) 2-Amino-benzenethiol (10mmol) and malononitrile (10mmol) were dissolved in 10ml of ethanol with a small amount of glacial acetic (10mmol) added. After stirring overnight, a yellow crystalline mass was recovered after filtration and drying, providing 82% of the theoretical yield of 2-cyanomethyl-benzothiazole (mp: 105-106°C).
ii) A 1:1 molar ratio of 2-cyanomethyl-benzothiazole and 2-aminophenol (10mmol were mixed uniformly, ground and transferred to a round bottomed flask. Polyphosphoric acid (approx. 80%, 20ml) was warmed until fluid and then poured into the flask. The flask was then placed in an oil bath at 185°C and heated in a nitrogen environment. After one hour, the flask was removed and the contents poured over crushed ice and stirred for one hour. The lumps which formed were broken down to yield a brown suspension which was filtered and the resulting solids washed with cold water until the washings were neutral. The product, 2-(2'-benzoxazolyl)-methyl-benzothiazole was then air dried.
iii) The final rigidized boron complex was synthesised from the 2-(2'-benzoxazolyl)-methyl-benzothiazole by first preparing the hydrobromide quaternary salt and then condensing this salt with boron trifluoride as in the previous Examples 1 and 3. The maximum absorptive wavelength for the compound was 388nm and the maximum emissive wavelength was 414nm, both measured in methanol. The compound was highly fluorescent in methanol.

### Example 5 Meso-acetyl-bis-(benzothiazolyl)methine boron difluoride

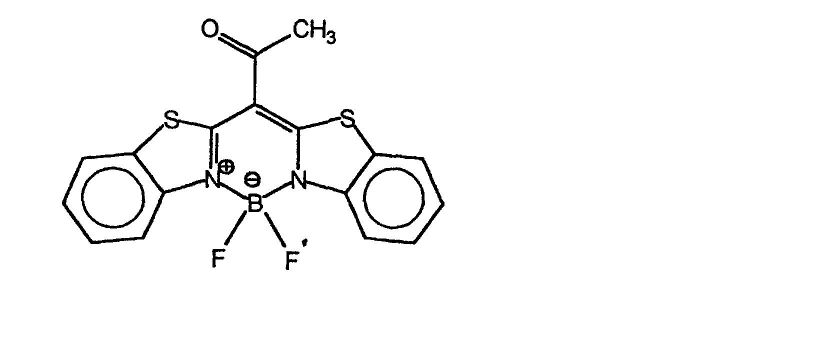

i) A sodium hydride slurry in mineral oil (80%, 30mg) was quickly transferred to a flame dried round bottomed flask fitted with a stirrer bar. Dry, freshly distilled tetrahydrofuran (THF) (4ml) was then added. In another flask, a weighed quantity of bis-benzothiazolyl methane (see Example 1) (0.28g, 1mmol) was dissolved in 1.5ml THF. This solution was added dropwise to the stirred sodium hydride slurry. Hydrogen gas evolved during the reaction was carefully vented off. After effervescence ceased, the reaction mixture was stirred for 0.5 hours.
ii) Acetyl chloride (0.078g, 1mmol) was added dropwise to the reaction mixture. Within minutes of the addition, the reaction mixture became turbid due to precipitation of sodium chloride. Stirring was continued for 2 hours, after which the solids were separated by filtration. The filtrate was evaporated to an oil which was then diluted with methanol (2ml) and left undisturbed for 0.5 hours, when a solid separated out. Further cooling yielded more solid. After filtration and drying, the product was obtained in approximately 30% yield.
iii) The hydrobromide quaternary salt of the meso-acetyl derivative above was prepared by the addition of hydrobromic acid. This was followed by condensation of the salt with boron difluoride by a procedure similar to that for Example 1 to yield meso-acetyl-bis-(benzothiazolyl)methine boron difluoride (27%). The maximum absorptive wavelength for the chromophore was 416nm measured in methanol. The chromophore was less fluorescent than the base benzothiazole chromophore.

### Example 6 Benzothiazolyl pyridylmonomethine boron difluoride

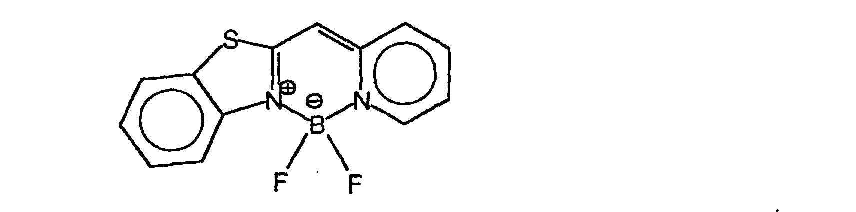

i) 2-Pyridylmethyl-benzothiazole was prepared by refluxing a 1:1 molar ratio of 2-cyanomethyl pyridine and 2-aminothiophenol in ethanol for 8 hours. Approximately 25ml ethanol was used for a 10mmol scale reaction. Following removal of the solvent, a yellow oil remained. The oil was dissolved in ether and washed with a 0.5M aqueous potassium hydroxide solution to remove unreacted thiol. The organic layer was then washed with a saturated sodium chloride solution, dried over magnesium sulphate and evaporated to again yield a yellow oil.
ii) The subsequent steps of quaternisation and condensation with boron trifluoride were similar to those used for the preparation of the compound of Example 1. The yield of benzothiazolyl pyridylmonomethine boron difluoride was 40-50% of theoretical. The maximum absorptive wavelength for the chromophore was 430nm and the maximum emissive wavelength was 476nm, both measured in methanol. The chromophore exhibited high fluorescence in methanol.

### Example 7 2-(2'-Benzothiazolyl)-quinoline methine boron difluoride

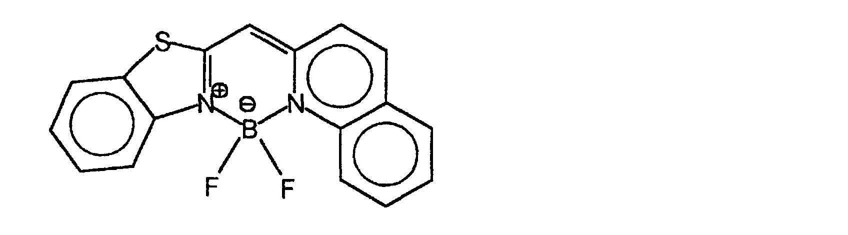

2-(2'-Benzothiazolylmethyl)-quinoline was prepared from 2-methyl benzothiazole and 2-chloroquinoline according to the procedure described in US Patent No.2541400. The resulting crude 2-(2'-benzothiazolylmethyl)-quinoline underwent the quaternization and condensation steps as in Example 1. 2-(2'-Benzothiazolyl)-quinoline methine boron difluoride exhibited a maximum absorptive wavelength of 480nm and a maximum emissive wavelength of 492nm, both measured in methanol. The chromophore exhibited a green fluorescence.

### Example 8 Protein Labelling

Approximately 20mg of bis-(carboxymethyl-benzoxazolyl)methine boron difluoride complex (bis-carboxymethyl dye of Example 3) and approximately 25mg of disuccinimidyl carbonate (DSC) were suspended in 250ml of anhydrous dimethyl formamide (DMF). The suspension was heated to 55°C to dissolve the bis-carboxymethyl dye and the DSC and the solution was maintained at that temperature for one hour. The bis-carboxymethyl dye and DSC reacted to form disuccinimidyl benzoxazolyl methine boron difluoride complex (Blue 1-OSu), a disuccinimidyl ester of Blue-1. A Sephadex G50 column was prepared with phosphate buffer solution (PBS). To 1mg sheep IgG protein in 400 microlitres CO₃⁻/HCO₃⁻ buffer solution (pH 9.6), was added 10-15 microlitres of the DMF reaction mixture containing the Blue 1-OSu. The protein/dye solution was vortexed for 10 minutes and the solution was then loaded onto the Sephadex column and eluted with PBS. The first fraction that eluted was the protein/dye conjugate which fluoresced in the blue region under a 365nm UV lamp.

An experiment was then carried out to label the sheep IgG protein at a higher dye-to-protein ratio (dye molecules per protein molecule). 1-2mg of the dried bis-carboxymethyl-OSu powder was added directly to 1mg of protein suspended in 400 microlitres of pH 9.6 buffer solution. The resulting dye/IgG conjugate was then purified on a Sephadex G50 column. It was estimated that the dye-to-protein ratio of the dye/protein conjugate was 2.7:1. The protein/dye conjugate in PBS was also tested for its emission spectrum and was determined to have a 380nm excitation maximum wavelength and an emission maximum wavelength range of 425nm. The quantum yield of the protein/dye conjugate was calculated to be 0.5.

### Example 9 6,6'-Disulpho-meso-carboxymethyl bis-(benzothiazolyl)methine boron difluoride

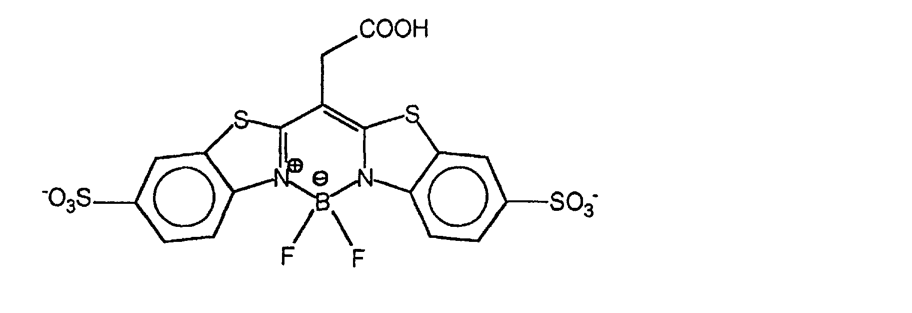

i) Sodium hydride slurry in mineral oil (80%, 30mg) was quickly transferred to a flame dried 25ml round bottom flask fitted with a stirrer bar. Dry, freshly distilled THF (4ml), was then added. In another flask, a weighed quantity of bis-benzothiazolyl methane (0.28g, 1mmol) was dissolved in 1.5ml dry THF. This solution was added dropwise to the stirred sodium hydride slurry. Hydrogen gas evolved during the reaction was carefully vented off. After the effervescence had ceased, the reaction mixture was stirred for 0.5hr.
   Methyl bromoacetate (0.153g, 1mmol) was added dropwise to the reaction mixture. Within minutes of addition, the reaction mixture turned turbid due to the formation of sodium bromide. Stirring was continued for 2hrs, after which time the solids were separated by filtration. The filtrate was evaporated to a green oil. The oil was diluted with methanol (2ml) and left undisturbed for 0.5hr, when a yellow/green solid separated out. Further cooling yielded more solid. After filtration and drying, 0. lg of solids were recovered (yield 28%).
ii) Methyl 3,3-bis-(benzothiazol-2-yl)propionate (0.176g, 0.5mmol) was dissolved in 8ml chloroform in a 25ml round bottom flask. Hydrobromic acid in acetic acid (30%, 50 microlitres) was added dropwise to the stirred solution. Within minutes a yellow precipitate formed. Stirring was continued for a further 0.5hr. Following filtration and drying, the hydrobromide salt was recovered as a bright yellow powder (0.12g, 55%).
iii) The hydrobromide salt from step ii) (1.0g, 2.3mmol) was suspended in dry toluene (15ml) in a 100ml round bottom flask fitted with a stirrer bar. N,N-Diisopropylethylamine (3ml) was added dropwise to the stirred suspension under a nitrogen atmosphere, the reaction mixture becoming clear. Boron trifluoride trietherate (5ml) was then carefully added to the reaction mixture, with the flask kept in an ice/water bath. After 4hr, stirring was stopped and the reaction mixture was filtered in a fume hood. After washing with water (100ml) and isopropanol (100ml) and drying, a yellow solid was recovered. Recrystallisation from isopropanol/chloroform yielded yellow crystals (0.25g, 27%) of meso-carboxymethyl-bis-(benzothiazolyl)methine boron difluoride (carboxymethyl-Blue 2 Dye).
iv) Carboxymethyl Blue 2 dye (0.2g) was suspended in 5ml methylene chloride. Chlorosulphonic acid (0.5ml) was injected dropwise into the suspension, following which it became clear. Chlorosulphonation was allowed to proceed for 18hr at room temperature. The reaction was then quenched by the addition of 10ml water. The organic layer was carefully separated. The product contained in the aqueous layer was subjected to base hydrolysis by the addition of sodium hydroxide (0.25g) and the solution stirred at room temperature for 20hrs. Completion of hydrolysis was indicated by dissolution of the product dye to give a clear blue fluorescing solution.
   The reaction product showed the presence of two compounds (Rf=0.8 and Rf=0.3) on reversed phase C-18 TLC when eluted in 10%methanol-water. The mixture was separated by means of reversed phase C-18 column chromatography using 10% methanol/water as eluent. Two fractions showing bright blue fluorescence were obtained, yielding the mono- and bis-sulphonated carboxymethyl Blue 2 dye (yields: 50mg, 10% and 120mg, 20% respectively).
v) The bis-sulphonated derivative was further used for the preparation of a succinimidyl ester and protein conjugation as follows. Approximately 5-10mg of the bis-sulphonated derivative prepared above was incubated with approximately 10mg of DSC in 0.25ml hexamethylphosphoramide (HMPA) and 50 microlitres of pyridine. The mixture was heated to 100°C with stirring and allowed to react for 0.25hr to provide the disuccinimidyl derivative. After cooling the product to room temperature, approximately 5-10 microlitres of this derivative were removed using a capillary tube and added to a freshly prepared solution of sheep IgG in PBS buffer (1mg in 400 microlitres) After 15 minutes, the dye/protein conjugate was separated from unreacted dye using a Sephadex G50 column.

### Example 10 α-Carboxymethyl-5,5'-disulpho-3,3'-ethylene-oxacyanine, methyl ester

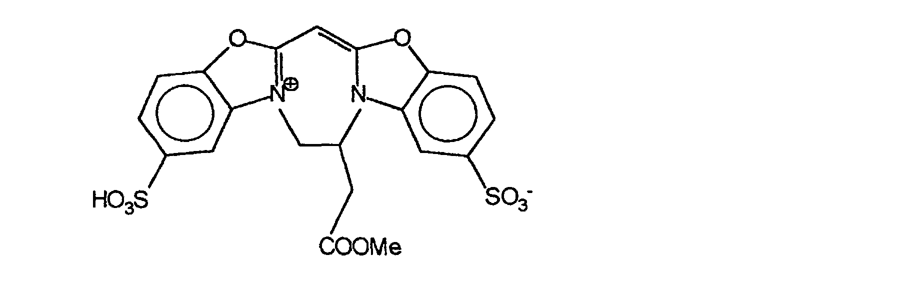

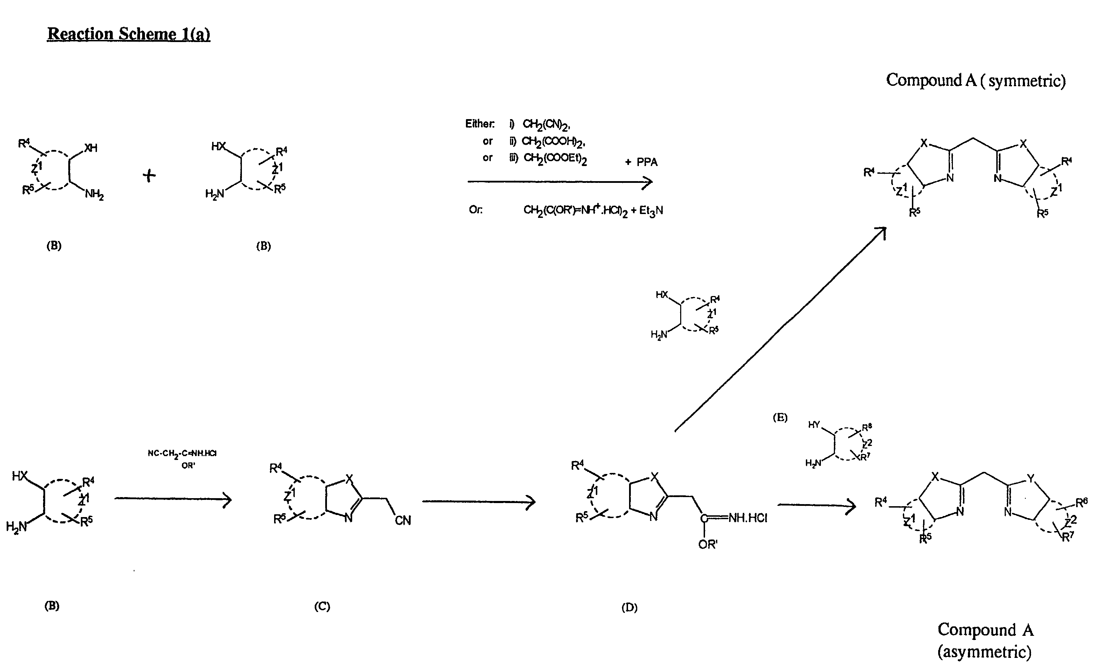

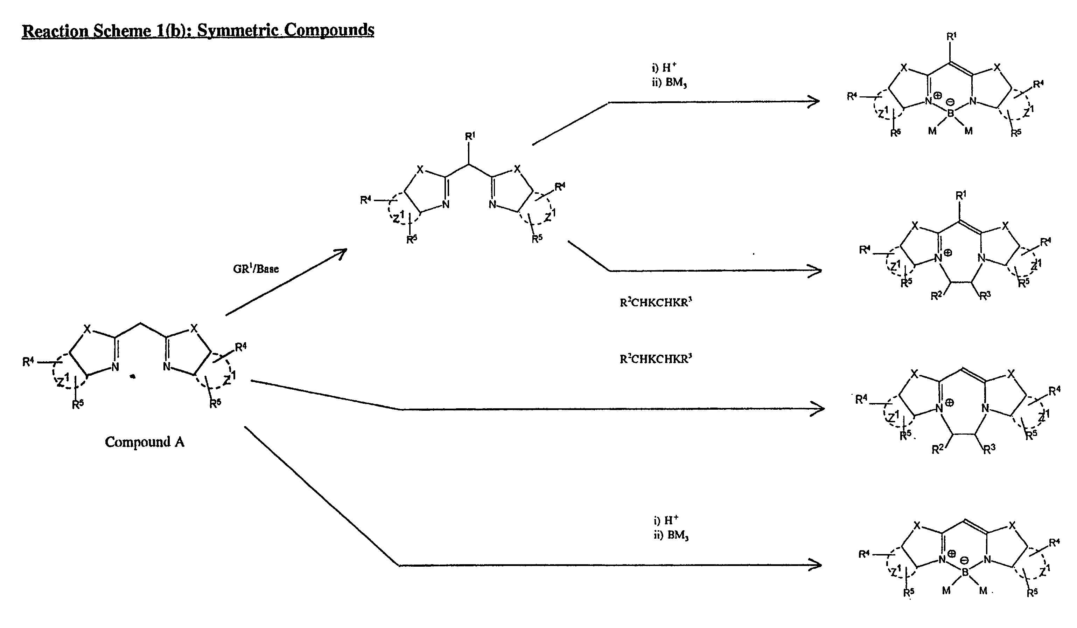

i) Ethyl bisimidate was prepared by a modification of the method of McElvain et al, (J.Amer.Chem.Soc., 71, 40, (1971)). Malononitrile (0.66g, 10mmol) was dissolved in dry dioxane (5ml). To this solution was added ethanol (0.92g, 20mmol). A 4M solution of HCI in dioxane (5ml) was injected in one portion into the malononitrile solution. The resulting mixture was stirred for 36 hrs at room temperature. The thick white slurry obtained was filtered, washed with three portions of 50ml of dry ether and dried under vacuum at room temperature for 1-2 hrs. The yield of ethyl bisimidate hydrochloride was 93%.
ii) To a suspension of 3-amino-4-hydroxybenzenesulphonic acid (18.9g, 0.1mol) in methanol (100ml) was added triethylamine (11.2g, 0.11 mol). The solution was concentrated to 50ml and cooled. A brown crystalline product of triethylamine salt was obtained which melted at 200°C with decomposition. The yield was 85-90% of theoretical.
   Triethylamine salt (29g, 0.1mol) was suspended in dry methanol (200ml). Freshly prepared ethyl bisimidate (11.5g, 0.05mol) was added to the suspension and the mixture was heated to reflux. Within 5 minutes the solution became clear. Heating was continued for 2 hours, after which the solution became turbid. The reaction mixture was then cooled and brown crystals of 5,5'-disulpho-3,3'-oxacyanine triethylamine salt were filtered off (yield 12g). The crystals melted at 170-175°C.
iii) The product obtained above (100mg, 0.2mmol) and methyl 3,4-di-para-tosyl butyrate (88mg, 0.2mmol) were thoroughly mixed and heated slowly at 185°C (oil bath temperature) for 20 minutes. The resulting dark brown mass was triturated with 0.2mmol triethylamine and isopropanol (20ml) until a free powder was obtained (100mg). The crude product showed three bright spots on C18 TLC in 10% methanol/water. The mixture was chromatographed on a C18 column with water/methanol mixture as eluent. The free acid with Rf=0.75 was recovered from the solvent to yield a silver coloured powder (yield 10mg).
   As shown in Fig.5 the UV spectrum of the compound showed an absorption maximum at 364nm and an emission maximum at 406nm when excited at 360nm. The quantum yield was 0.8 with quinine sulphate as the standard. This free acid dye was used to prepare the succinimidyl ester without further purification.
iv) The acid obtained from above was dissolved in 100 microlitres of dry DMF containing 10 microlitres of pyridine. Excess (10mg) DSC was added and the mixture was heated at 65-70°C for 2 hours under a nitrogen atmosphere. After completion of the reaction, dry diethyl ether (50ml) was added. The precipitated active ester was filtered and dried in a vacuum for 1 hour.

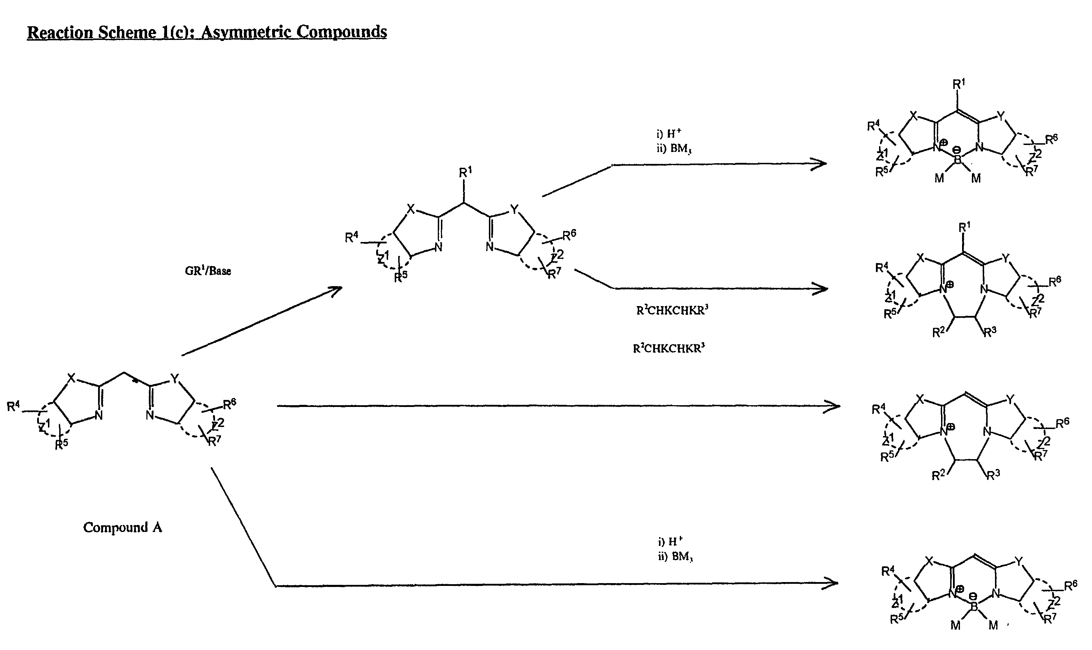

The dried active ester (approximately 1mg) was dissolved in DMF (50 microlitres) and 10 microlitres of this stock solution was allowed to react for 30 minutes with 1mg of sheep IgG protein dissolved in 250ml carbonate-bicarbonate buffer (pH 9.4). The dye antibody conjugate was separated from unreacted dye on a size exclusion column (Sephadex G50) using PBS solution (pH 7) as eluent. The absorption and emission spectra of the dye-antibody conjugate are shown in Fig.6. The protein absorbed at 280nm (0.1387 absorbance units) and the dye absorbed at 372nm (0.04257 absorbance units).

### Example 11 Meso-5-carboxypentyl-3,3'-ethylenethiacyanine

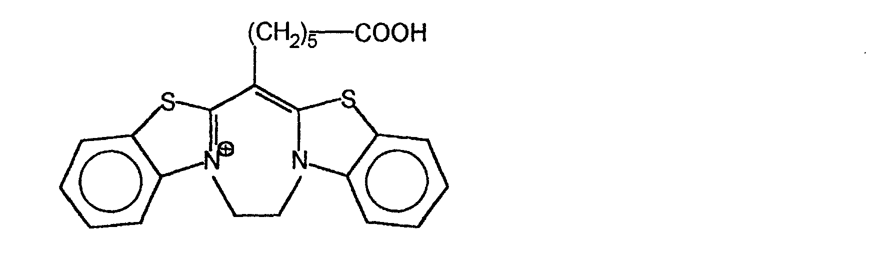

i) A sodium hydride slurry in mineral oil (80%, 30mg) was quickly transferred to a flame dried 25ml round bottom flask fitted with a stirrer bar. Dry, freshly distilled THF (4ml), was then added. In another flask, a weighed quantity of bis-benzothiazolyl methane (0.28g, 1mmol) was dissolved in 1.5ml dry THF. This solution was added dropwise at room temperature to the stirred sodium hydride slurry. Hydrogen gas evolved during the reaction was carefully vented off. After the effervescence had ceased, the reaction mixture was stirred for 0.5hr.
   Methyl iodohexanoate (0.256g, 1mmol) was added dropwise to the reaction mixture. Within minutes of addition, the reaction mixture turned turbid due to the formation of sodium iodide. Stirring was continued for 2hrs, after which time the solids were separated by filtration. The filtrate was evaporated to a green oil. The oil was diluted with methanol (2ml) and left undisturbed for 0.5hr, when a yellow/green solid separated out. Further cooling yielded more solid. After filtration and drying, 0.1g of the product, meso-bis-(benzothiazolyl)methane-hexanoic acid, methyl ester, was recovered (yield 28%).
ii) The product from step i) (100mg, 0.24 mmol) and ethylene glycol di-p-tosylate (100mg, 0.27mmol) were thoroughly mixed and heated at 185°C (oil bath temperature) for 20 minutes. The resulting dark brown mass was triturated with 0.2 mmol of triethylamine until a free powder was obtained (50mg). Following hydrolysis of the methyl ester with sodium hydroxide, the crude product was chromatographed on a C18 reversed phase column with water/methanol as eluent to give the desired free acid. The UV spectrum of this compound in water, 0.1N HCl and 0.1N NaOH showed an absorption maximum at 454nm and an emission maximum at 472nm when excited at 418nm. The quantum yield was 0.8 with quinine sulphate as the standard. In separate testing using PBS as the solvent, the compound showed an absorbance maximum of 454nm and an emission maximum at 472nm. A quantum yield of 0.28 was recorded using Coumarin 30 in ethanol as the reference standard.
   Meso-5-carboxypentyl-3,3'-ethylenethiacyanine was converted to its succinimidyl ester and conjugated to sheep IgG protein. In PBS, the conjugate was found to have an absorbance maximum of 456nm, an emission maximum of 472nm and a quantum yield of 0.15 using Coumarin 30 in ethanol as the reference standard.
iii) Meso-5-carboxypentyl-3,3'-ethylenethiacyanine was sulphonated using the following procedure. The dye (100mg) was dissolved in a mixture of concentrated sulphuric acid (1ml) and acetic anhydride (1ml) and heated to 140°C for 1 hour. The mixture was cooled and the dark brown mass triturated with acetone (50ml). The resulting solid was filtered and chromatographed on a reversed phase C18 column, with 5% methanol/water as eluent, Rf=0.3.

### Example 12 3,3'-Ethylene-6-sulphothia-5'-carboxymethyl-6'-sulpho-oxa monomethine cyanine

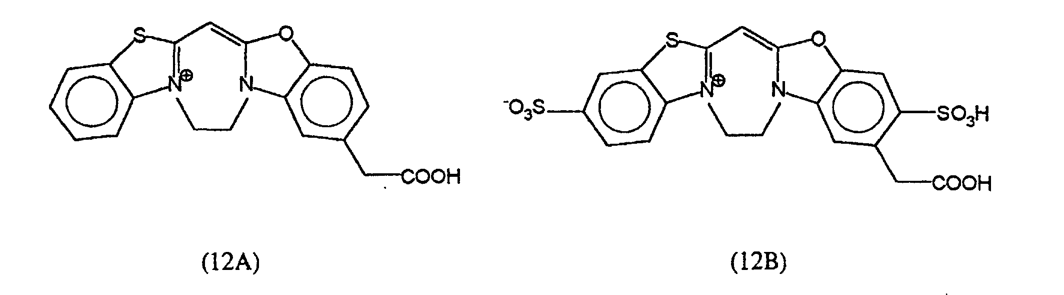

i) To a magnetically stirred solution of 4-hydroxyphenylacetic acid (100g, 0.65mol) in glacial acetic acid (250ml) cooled in an ice bath, was added dropwise, a mixture of concentrated nitric acid (40ml) and glacial acetic acid (80ml) over 20 minutes. Stirring was continued for 1 hour at 5°C and then 1 hour at room temperature. The solution turned yellowish brown during the addition. The viscous mass was filtered and washed with cold water and dried at room temperature. The product was crystallised from methanol to yield bright yellow crystals (72g, 56%), mp. 146-7°C; ¹H NMR (CDCl₃, δ): 8.0 (1H, s, 2-H); 7.5 (1H, d, J=7Hz, 6H); 7.1 (1H, d, J=7Hz, 5-H); 3.6 (2H, s, CH₂).
ii) To a stirred solution of sodium hydroxide (1g) in 40ml of water in a round bottom flask, was added 3-nitro-4-hydroxyphenylacetic acid from step i) (4.93g, 0.025mol). The solid dissolved at once giving an orange coloured solution. 10% Pd/Charcoal (50mg) was added, followed by the dropwise addition of hydrazine monohydrate (3.25ml). After the completion of the addition (5 minutes), the mixture was heated at 60°C for 1.5hr. The temperature was raised to reflux and heating continued for an additional 0.5hr. The solution appeared clear with Pd/C suspended in it. The solution was filtered hot, concentrated to half volume and then cooled. The solution was then acidified to pH 4-5 with acetic acid. The white precipitate, 3-amino-4-hydroxyphenylacetic acid, which separated after cooling was filtered off and washed with ethanol, mp.225-7°C (yield 3.4g, 30%). ¹H NMR (D₂O, δ) 7.9 (1H, d, J=7Hz, 6-H); 7.8 (s, 1H, 2-H); 7.65 (1H, d, J=7Hz, 5-H); 3.4 (2H, s, CH₂COOH).
iii) 2-Cyanomethylbenzothiazole was prepared according to the method of Saito et al, Synthesis, 210-11, (1983), as given in Example 4, Step i).
iv) A mixture of 2-cyanomethylbenzothiazole (1.74g, 0.01mol) and sodium methoxide (0.5g, 0.01mol) in anhydrous methanol (50ml) was stirred at room temperature for 15hr. The orange powder obtained was used in the next stage without separation. Acetic acid was added to the mixture to neutralise sodium methoxide. 3-Amino-4-hydroxyphenylacetic acid (1.67g, 0.01mol) was added and the mixture heated to reflux. After 4 hours methanol was distilled off and the residue (3.4g) was purified by flash chromatography on a silica gel column (50g) using chloroform/methanol as eluent. Yellowish green crystals were obtained from ethanol, mp.182-4°C (yield 0.75g, 23%). ¹H NMR (DMSO-d₆, δ) 8.1 ( 1H, d, J=7Hz), 7.9 (1H, d, J=7Hz), 7.62 (1H, D, J=7Hz), 7.6 (1H, s), 7.4-7.55 (2H, m), 7.3 (1H, d, J=7Hz), 5.0 (2H, s, CH₂-bridge), 3.7 (2H, s, CH₂COOH).
v) 324mg (1mmol) of the acid obtained from step iv) and 370mg (1mmol) of ethylene glycol di-p-toluenesulphonate were heated together for 4hr at 170°C. The resulting yellow solid product was cooled and to it was added acetone (100ml) followed by triethylamine (2ml). The solution was evaporated to dryness and the residue washed with ether to remove excess triethylamine. The solid dark brown mass was then dissolved in methanol. Sodium iodide dissolved in 10ml hot methanol was added to convert the 3,3'-ethylene-cyanine p-sulphonate to the iodide. The solvent was removed and the entire mass dissolved in a solution of 10% methanol in chloroform, followed by flash chromatography on silica gel using chloroformmethanol as eluent. Two yellow products appeared very bright in UV light and were isolated. The product from fraction A (20mg, Rf=0.3, silica gel using 10% methanol/chloroform) was not characterised. The residue from fraction B, after evaporation was characterised as the desired dye, 3,3'-ethylene-thia-(5'-carboxymethyloxa)monomethine cyanine (Compound 12A), (yield 150mg, Rf=0.1, silica gel using 50% methanol/chloroform). It was purified on a reversed phase C18 column, using water methanol mixture as the eluent. ¹H NMR (DMSO-d6, δ) 8.2 (1H, d, J=7Hz), 7.9 (1H, D, J=7Hz), 7.45-7.6 (3H, m), 7.4 (1H, t), 7.3 (1H, d, J=7Hz), 6.5 (1H, s, CH-bridge), 4.9-4.7 (4H, broad m, CH₂-CH₂), 3.4 (2H, s, CH₂COOH). UV (methanol) λₘₐₓ 410nm, ε 61000, Emₘₐₓ 420nm, φ 0.24 in water and 0.82 in methanol, based on Coumarin 30 as standard.
vi) 3,3'-Ethylene-thia-(5'-carboxymethyloxa)monomethine cyanine (Compound 12A) (100mg) was dissolved in a mixture of concentrated sulphuric acid (1ml) and acetic anhydride (1ml) and heated to 140°C for 1hr. The mixture was cooled and the dark brown mass was triturated with acetone (50ml). The solution was filtered and the solid obtained was chromatographed on a reversed phase C18 column, with water as solvent, to give 3,3'-ethylene-6-sulphothia-5'-carboxymethyl-6'-sulpho-oxa monomethine cyanine (Compound 12B). (Rf=0.8, C18, water). ¹H NMR (D₂O, δ) 8.2 (1H, s, 7-H), 8.1 (1H, s, 7'-H), 7.95 (1H, d, J=7Hz, 5-H), 7.75 (1H, d, J=7Hz, 4-H), 7.45 (1H, s, 4'-H), 6.3 (1H, s, CH-bridge), 4.9-4.7 (4H, broad m, CH₂-CH₂, merged in a water signal), 4.0 (2H, s, CH₂COOH). UV (methanol) λₘₐₓ 414nm, ε 70000, Emₘₐₓ 420nm, φ 0.60 (water) based on Coumarin 30 as standard.
vii) Dye/protein conjugation experiments were carried out on the non-sulphonated dye (Compound 12A) and sulphonated dye (Compound 12B). Both compounds were converted to their succinimidyl esters respectively, according to the method described in Mujumdar et al, Bioconjugate Chem., 4, 105, (1993). The dye (1mg) was dissolved in 100 microlitres dry DMF containing 10 microlitres pyridine. DSC (7mg) was added to this mixture and heated at 65-70°C for two hours under a nitrogen atmosphere. After completion of the reaction, the solvents were removed under reduced pressure at 50oC. The dried succinimidyl ester was dissolved in 100 microlitres dry DMF and 10 microlitres of this stock solution was allowed to react for 30 minutes with 1mg of sheep IgG dissolved in 250 microlitres of carbonate/bicarbonate buffer (pH 9.4). The dye antibody conjugate was separated from unreacted dye by size exclusion chromatography (Sephadex G50) using PBS (pH 7) as eluent. The following spectral data were obtained for each of the conjugated and unconjugated compounds (12A) and (12B) in various solvents, (see Table 4).

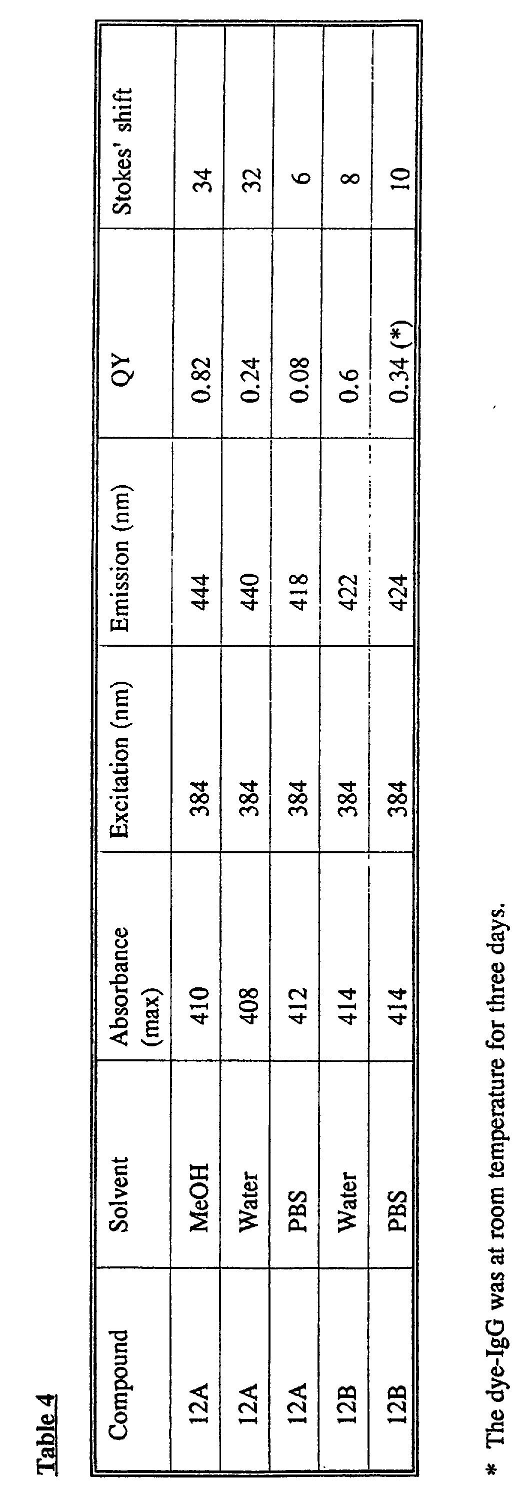

The dye (12A) was also used in experiments to tag DNA. 5-Aminopropargyl-2'-deoxycytidine-5'-triphosphate was incorporated into the sequence of DNA by a standard nick-translation reaction. The resulting DNA containing aliphatic amino groups was purified by ethanol precipitation, dissolved in borate-EDTA buffer and stored at -20°C. Fluorescent DNA was formed by reacting this amino-DNA (approx 1 microgram) with the N-hydroxysuccinimidyl ester of 12A. 1 microgram amino-propargyl DNA in 25 microlitres of buffer was diluted with 25 microlitres formamide, heated to 77°C for 5 minutes to denature DNA into single stranded form, then immediately chilled on ice to inhibit reformation of double stranded DNA. This cold solution was diluted with an equal volume of carbonate buffer (0.1M, pH 9.2). The active ester of 12A (approx 10mmoles in 1 microlitre DMF) was added to the DNA and the mixture was incubated at room temperature for 1 hour. The DNA was precipitated with ammonium acetate/ethanol, washed twice with ice-cold 70% ethanol, then redissolved in TRIS-EDTA buffer. The fluorescent DNA product was analysed by agarose gel electrophoresis and visualised using standard UV trans-illumination.

### Examples 13 and 14

Using the synthetic pathways of the present invention, the following compounds, 13 and 14, were prepared.

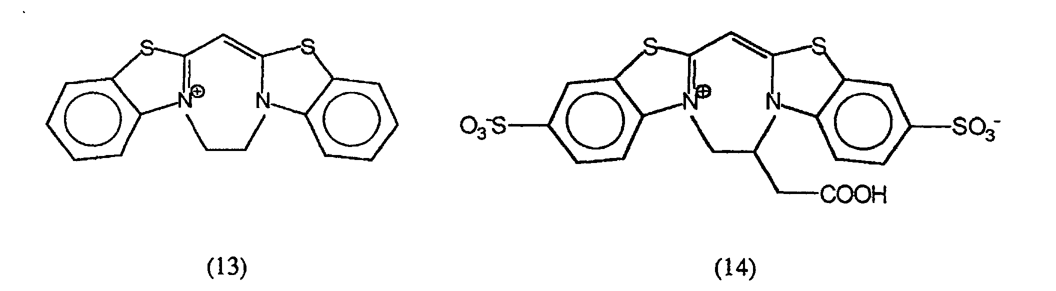

The compounds exhibited the following spectral properties in the indicated solvents (see Table 5). Compound 14 was also conjugated to sheep IgG protein and exhibited the indicated spectral preperties. The quantum yields were determined using Coumarin 30 in ethanol as the reference standard.

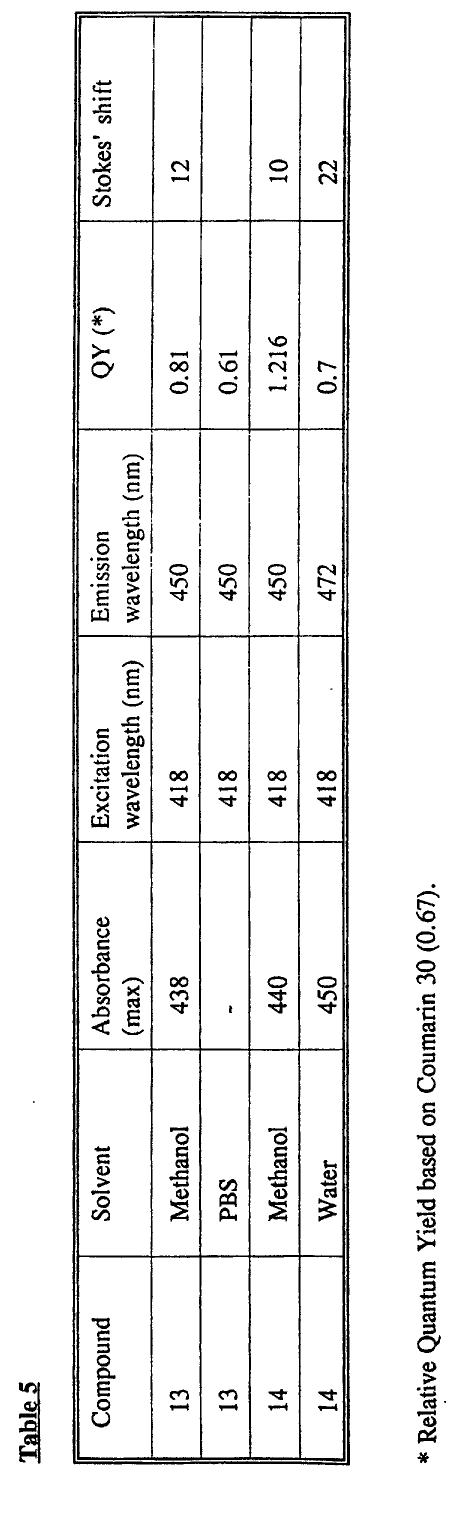

### Example 15 Covalent Labelling of a Glass Surface

Alkoxysilanes are known to react with glass surfaces. One such reagent, 3-aminopropyltrimethoxysilane, is known to coat porous glass beads to form the aminopropyl derivative of the glass for use as an absorbant for affinity chromatography. See, Biochem.Biophys.Act., 212, 1, (1970); J.Chromatography, 97, 39, (1974). This procedure was adapted to stain glass slides with fluorescent dye compounds of the present invention.

Gold Seal Microslides (Becton-Dickinson) were washed with distilled water and with acetone. The slides were then treated with a 10% (v/v) solution of 3-aminopropyltrimethoxysilane (Sigma Chemicals) in xylene for 30 minutes. The slides were then rinsed in absolute ethanol to remove the xylene, rinsed in water and air dried. A solution (approximately 200 microlitres), of carbonate/bicarbonate buffer (pH 9.4) was placed at the centre of each slide, together with 20 microlitres of a solution of a dye of the present invention dissolved in DMF (approximately 2mg dye/100 microlitres DMF). The dye compound used was the following succinimidyl ester monomethine cyanine compound:

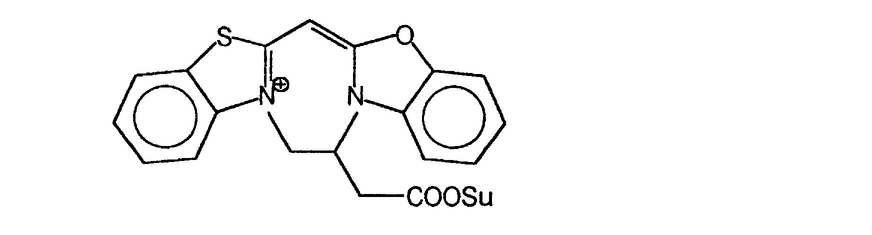

The succinimidyl ester derivative was prepared by the method generally described in US Patent No.5268486. The slides were incubated for 20 minutes before being rinsed with distilled water. In this way, the succinimidyl ester dye compound was attached covalently to the surface of the glass slide. The presence of the covalently-attached dye on the glass surface was detected by fluorescence spectrophotometry.

### Figures

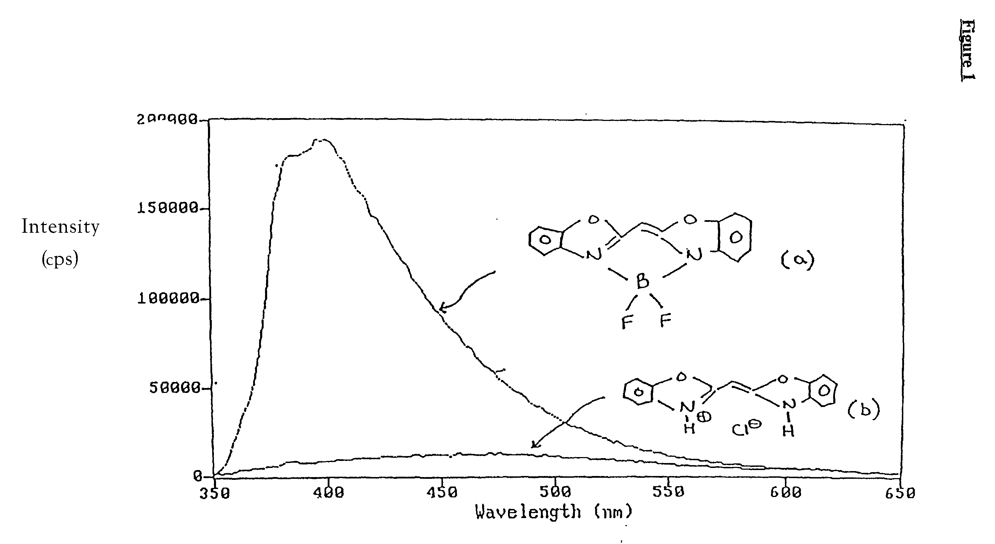

Figure 1 is a plot of relative fluorescence intensity versus wavelength for bis-benzoxazolylmethine boron difluoride complex of the present invention and bis-benzoxazolylmethine hydrochloride;
Figure 2 is a plot of the absorption and emission spectra of bis-benzothiazolylmethine boron difluoride compound of the present invention; and
Figure 3 is a plot of the absorption and emission spectra of bis-benzoxazolylmethine boron difluoride compound of the present invention.
   The increase in fluorescence derived from rigidization of the heterocycles of the compounds of the present invention is demonstrated in Figure 1 which shows the relative fluorescence spectra in methanol of bis-benzoxazolylmethene boron difluoride complex (curve a) and bis-benzoxazolylmethene boron difluoride hydrochloride (curve b), both excited at 348nm. The quantum yield of the boron-rigidized complex is several fold greater than the unrigidized dye.

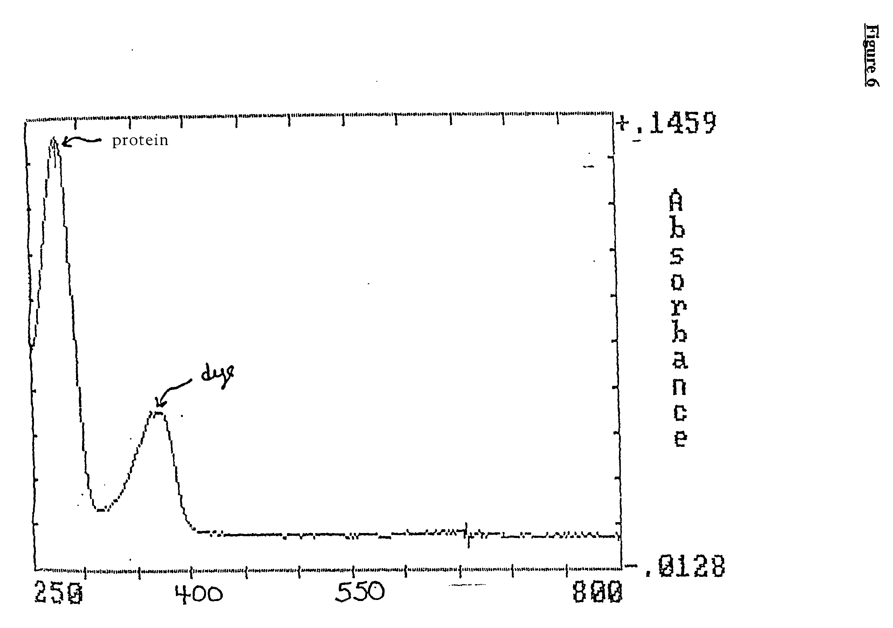

Figure 4 is a plot of the relative fluorescence spectra of an ethylene-rigidized benzoxazolylmethine complex of the present invention (curve a), 5,5'-disulpho-3,3'-ethylene-oxacyanine, a non-rigidized N,N'-dimethyl-di-2-benzoxazolyl methane in glycerol (curve b) and in water (curve c),,all samples being excited at 364nm.
Figure 5 is a plot of the UV absorption spectrum and emission spectrum for the compound of Example 10.
Figure 6 is a plot of the UV absorption spectrum of the compound of Example 10 conjugated to IgG sheep antibody.

## Claims

1. A compound of formula: 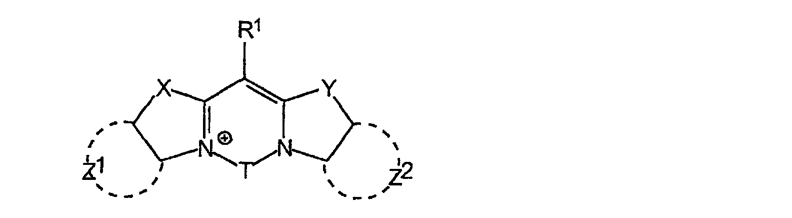 optionally substituted by groups R⁴ to R⁷, or by groups R² to R⁷ when T contains carbon atoms, wherein R² and R³ are attached to the carbon atoms of T and R⁴, R⁵, R⁶ and R⁷ are attached to the rings containing X and Y or, optionally, are attached to atoms of the Z¹ and Z² ring structures;
groups R² to R⁷ which are the same or different are selected from -R⁹ and -L-R⁹ where R⁹ is selected from neutral groups that reduce water solubility, polar groups that increase water solubility, functional groups that can be used in labelling reactions, reactive groups, electron donating and withdrawing groups that shift the absorption and emission wavelengths of the fluorescent molecule, lipid and hydrocarbon solubilising groups, and L is selected from the group consisting of a straight or branched C₁₋₂₆ alkyl chain, a C₂₋₂₀ monoether or polyether and a C₂₋₂₀ atom chain containing up to four secondary amide linkages;
R¹ is selected from hydrogen, aryl, heteroaryl, cyano, nitro, aldehyde, halogen, hydroxy, alkyl groups of twenty-six carbon atoms or less, amino, quaternary amino, acetal, ketal, phosphoryl, sulphydryl, water-solubilizing groups, and -(CH₂)ₙQ where l<n<26 and Q is selected from amino, substituted amino, quaternary amino, aldehyde, acetal, ketal, halo, cyano, aryl, heteroaryl, hydroxyl, sulphonate, sulphate, carboxylate, amide, nitro, and groups reactive with amino, hydroxyl, aldehyde, phosphoryl, or sulphydryl groups;
T is a linking group such that: 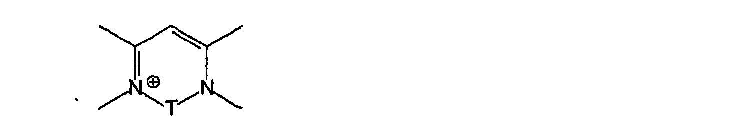 is a six or seven membered ring;
X and Y may be the same or different and are selected from bis-substituted carbon, oxygen, sulphur, selenium, CH=CH, and N-W wherein N is nitrogen and W is selected from hydrogen, a group -(CH₂)ₙR⁸ where n is an integer from 1 to 26 and R⁸ is selected from hydrogen, amino, aldehyde, acetal, ketal, halo, cyano, aryl, heteroaryl, hydroxyl, sulphonate, sulphate, carboxylate, substituted amino, quaternary amino, nitro, primary amide, substituted amide, and groups reactive with amino, hydroxyl, carbonyl, phosphoryl, and sulphydryl groups;
one of groups Z¹ and Z² represents a bond or the atoms necessary to complete one, two fused or three fused aromatic rings each ring having five or six atoms, selected from carbon atoms and, optionally, no more than two oxygen, nitrogen and sulphur atoms and the other group Z¹ or Z² represents the atoms necessary to complete one, two fused or three fused aromatic rings each ring having five or six atoms, selected from carbon atoms and, optionally, no more than two oxygen, nitrogen and sulphur atoms;
provided that at least one of R¹ to R⁷ comprises a reactive group for covalent reaction with a functional group on a target material or comprises a functional group for covalent reaction with a reactive group on a target material.

2. A compound of formula: 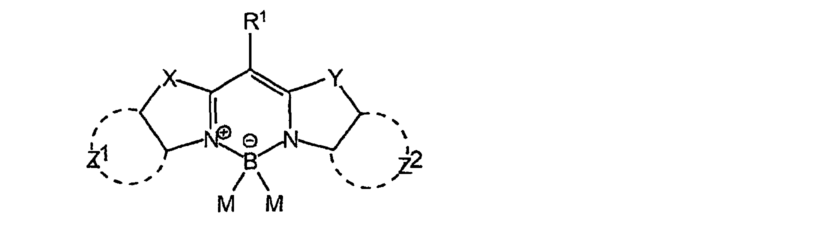 optionally substituted by groups R⁴ to R⁷, wherein R⁴, R⁵, R⁶ and R⁷ are attached to the rings containing X and Y or, optionally, are attached to atoms of the Z¹ and Z² ring structures, and
wherein M is selected from F and Cl;
groups R⁴ to R⁷ which are the same or different are selected from -R⁹ and -L-R⁹ where R⁹ is selected from neutral groups that reduce water solubility, polar groups that increase water solubility, functional groups that can be used in labelling reactions, reactive groups, electron donating and withdrawing groups that shift the absorption and emission wavelengths of the fluorescent molecule, lipid and hydrocarbon solubilising groups, and L is selected from the group consisting of a straight or branched C₁₋₂₆ alkyl chain, a C₂₋₂₀ monoether or polyether and a C₂₋₂₀ atom chain containing up to four secondary amide linkages;
R¹ is selected from hydrogen, aryl, heteroaryl, cyano, nitro, aldehyde, halogen, hydroxy, alkyl groups of twenty-six carbon atoms or less, amino, quaternary amino, acetal, ketal, phosphoryl, sulphydryl, water-solubilizing groups, and -(CH₂)ₙQ where l<n<26 and Q is selected from amino, substituted amino, quaternary amino, aldehyde, acetal, ketal, halo, cyano, aryl, heteroaryl, hydroxyl, sulphonate, sulphate, carboxylate, amide, nitro, and groups reactive with amino, hydroxyl, aldehyde, phosphoryl, or sulphydryl groups;
X and Y may be the same or different and are selected from bis-substituted carbon, oxygen, sulphur, selenium, CH=CH, and N-W wherein N is nitrogen and W is selected from hydrogen, a group -(CH₂)ₙR⁸ where n is an integer from 1 to 26 and R⁸ is selected from hydrogen, amino, aldehyde, acetal, ketal, halo, cyano, aryl, heteroaryl, hydroxyl, sulphonate, sulphate, carboxylate, substituted amino, quaternary amino, nitro, primary amide, substituted amide, and groups reactive with amino, hydroxyl, carbonyl, phosphoryl, and sulphydryl groups;
one of groups Z¹ and Z² represents a bond or the atoms necessary to complete one, two fused or three fused aromatic rings each ring having five or six atoms, selected from carbon atoms and, optionally, no more than two oxygen, nitrogen and sulphur atoms and the other group Z¹ or Z² represents the atoms necessary to complete one, two fused or three fused aromatic rings each ring having five or six atoms, selected from carbon atoms and, optionally, no more than two oxygen, nitrogen and sulphur atoms;
provided that at least one of R¹ and R⁴ to R⁷ comprises a reactive group for covalent reaction with a functional group on a target material or comprises a functional group for covalent reaction with a reactive group on a target material.

3. A compound of the formula: 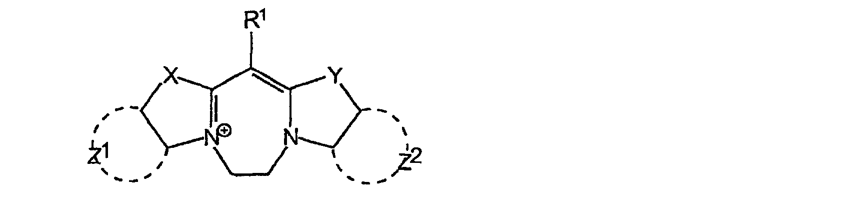 optionally substituted by groups R² to R⁷ where groups R² to R³ are attached to the ethylene linking group, 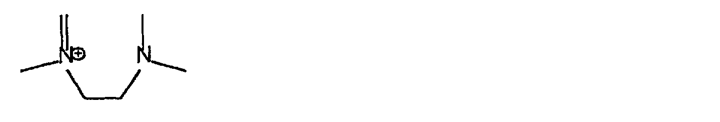 and groups R⁴ to R⁷ are attached to the rings containing X and Y or are optionally attached to atoms of the Z¹ and Z² ring structures;
groups R² to R⁷ which are the same or different are selected from -R⁹ and -L-R⁹ where R⁹ is selected from neutral groups that reduce water solubility, polar groups that increase water solubility, functional groups that can be used in labelling reactions, reactive groups, electron donating and withdrawing groups that shift the absorption and emission wavelengths of the fluorescent molecule, lipid and hydrocarbon solubilising groups, and L is selected from the group consisting of a straight or branched C₁₋₂₆ alkyl chain, a C₂₋₂₀ monoether or polyether and a C₂₋₂₀ atom chain containing up to four secondary amide linkages;
R¹ is selected from hydrogen, aryl, heteroaryl, cyano, nitro, aldehyde, halogen, hydroxy, alkyl groups of twenty-six carbon atoms or less, amino, quaternary amino, acetal, ketal, phosphoryl, sulphydryl, water-solubilizing groups, and -(CH₂)ₙQ where l<n<26 and Q is selected from amino, substituted amino, quaternary amino, aldehyde, acetal, ketal, halo, cyano, aryl, heteroaryl, hydroxyl, sulphonate, sulphate, carboxylate, amide, nitro, and groups reactive with amino, hydroxyl, aldehyde, phosphoryl, or sulphydryl groups;
X and Y may be the same or different and are selected from bis-substituted carbon, oxygen, sulphur, selenium, CH=CH, and N-W wherein N is nitrogen and W is selected from hydrogen, a group -(CH₂)ₙR⁸ where n is an integer from 1 to 26 and R⁸ is selected from hydrogen, amino, aldehyde, acetal, ketal, halo, cyano, aryl, heteroaryl, hydroxyl, sulphonate, sulphate, carboxylate, substituted amino, quaternary amino, nitro, primary amide, substituted amide, and groups reactive with amino, hydroxyl, carbonyl, phosphoryl, and sulphydryl groups;
one of groups Z¹ and Z² represents a bond or the atoms necessary to complete one, two fused or three fused aromatic rings each ring having five or six atoms, selected from carbon atoms and, optionally, no more than two oxygen, nitrogen and sulphur atoms and the other group Z¹ or Z² represents the atoms necessary to complete one, two fused or three fused aromatic rings each ring having five or six atoms, selected from carbon atoms and, optionally, no more than two oxygen, nitrogen and sulphur atoms;
provided that at least one of R¹ to R⁷ comprises a reactive group for covalent reaction with a functional group on a target material or comprises a functional group for covalent reaction with a reactive group on a target material.

4. A compound according to claims 1, 2 or 3 wherein R⁹ is selected from:
- neutral groups selected from the group consisting of hydrogen and halogen atoms;
- polar groups selected from the group consisting of amide, sulphonate, sulphate, phosphate, quaternary ammonium, guanidinium, hydroxyl, phosphonate;
- functional groups selected from the group consisting of optionally substituted amino, azido, hydroxyl, sulphydryl, imidazole, carboxyl and carbonyl;
- reactive groups selected from the group consisting of succinimidyl ester, isothiocyanate, isocyanate, anhydride, haloacetamide, maleimide, sulphonyl halide, phosphoramidite, acid halide, acyl azide, alkylimidate, hydrazide, arylimidate, hydroxylamines, carbodiimides;
- electron donating and withdrawing groups selected from the group consisting of amide, cyano, nitro, alkoxy, styryl, aryl and heteroaryl groups;
- lipid and hydrocarbon solubilising groups selected from the group consisting of alkyl, aryl and aralkyl groups; and
L is selected from the group consisting of a straight or branched C₁₋₂₆ alkyl chain, a C₂₋₂₀ monoether or polyether and a C₂₋₂₀ atom chain containing up to four secondary amide linkages.

5. A compound according to claim 1 selected from:
i) 6,6'-Disulpho-meso-carboxymethyl bis-(benzothiazolyl)methine boron difluoride
ii) α-Carboxymethyl-5,5'-disulpho-3,3'-ethylene-oxacyanine
iii) 3,3'-Ethylene-6-sulphothia-5'-carboxymethyl-6'-sulphooxa monomethine cyanine
iv) 5-Carboxymethyl-bis-(benzoxazolyl)methine boron difluoride
v) α-Carboxymethyl-3,3'-ethylene thiacyanine
vi) *meso*-Carboxymethyl-benzoxazolyl-benzothiazolyl monomethine boron difluoride

6. A method for producing a compound according to any one of claims 1 to 5 comprising reacting a compound of formula (A), or a protonated form thereof, 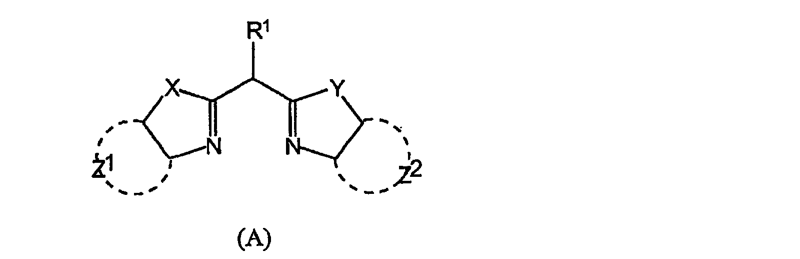 optionally substituted by groups R⁴ to R⁷, wherein R¹ and R⁴ to R⁷, X, Y, Z¹ and Z² are hereinbefore defined, with a compound suitable for the formation of linkage T, wherein T is as hereinbefore defined.

7. A compound of formula: 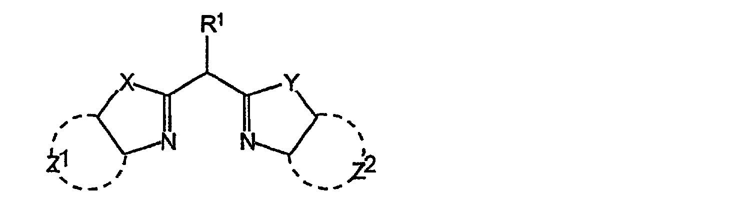 or a protonated form thereof;
optionally substituted by groups R⁴ to R⁷ wherein R⁴, R⁵, R⁶ and R⁷ are attached to the rings containing X and Y or, optionally, are attached to atoms of the Z¹ and Z² ring structures and wherein R¹, R⁴ to R⁷, X, Y, Z¹ and Z² are hereinbefore defined;
provided that at least one of R¹ to R⁷ comprises a reactive group for covalent reaction with a functional group on a target material or comprises a functional group for covalent reaction with a reactive group on a target material.

8. A method of imparting fluorescent properties to a non-polar material, the method comprising the steps of dissolving in the non-polar material the compound as recited in any one of claims 1 - 5, wherein at least one groups R¹ to R⁷ is an uncharged group.

9. A method of imparting fluorescent properties to a polar material, the method comprising the steps of dissolving in the polar material a compound as claimed in any one of claims 1-5 wherein at least one of the R-groups is selected from the group consisting of charged groups and polar groups.

10. A method for imparting fluorescent properties to a target material, the method comprising the steps of incubating:
i) a target material having at least one functional group selected from the group consisting of amino, hydroxyl, phosphoryl, carbonyl and sulphydryl groups; or having at least one reactive group that can covalently bond with said at least one functional group, and;
ii) an amount of the fluorescent compound as claimed in any one of claims 1-5 wherein at least one of the R-groups is a functional group selected from the group consisting of amino, hydroxyl, phosphoryl, carbonyl and sulphydryl; or wherein at least one of the R-groups is a reactive group that can covalently bond with said at least one functional group;
for a period of time sufficient to permit said at least one functional or reactive group of said fluorescent compound to covalently bond to said at least one reactive or functional group of said target material.

11. A method for the determination of the sequence of a nucleic acid said method comprising the steps of:
i) providing a sample of said nucleic acid to be sequenced, a primer nucleic acid sequence which is complementary to at least a part of said nucleic acid to be sequenced, a supply of deoxynucleotides and at least one dideoxynucleotide for terminating the sequencing reaction, and a polymerase;
ii) performing nucleic acid chain extension and chain termination reactions;
iii) separating the oligonucleotide fragments according to size;
**characterised in that** one or more of said dideoxynucleotides or said primer nucleic acid sequence is labelled with a compound as claimed in any one of claims 1-5.

## Patentansprüche

1. Verbindung der Formel: 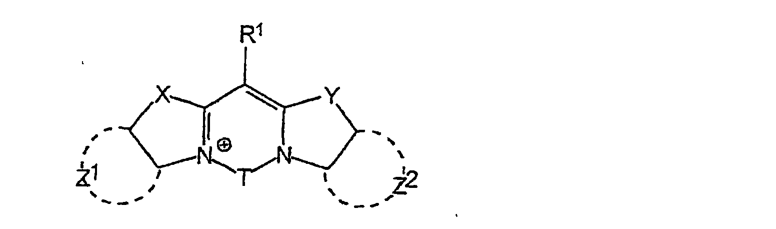 wahlweise substituiert durch Gruppen R⁴ bis R⁷, oder durch Gruppen R² bis R⁷, wenn T Kohlenstoffatome enthält, worin R² und R³ an die Kohlenstoffatome von T gebunden sind und R⁴, R⁵, R⁶ und R⁷ an die X und Y enthaltenden Ringe gebunden sind, oder wahlweise an Atome der Z¹- und Z²-Ringstrukturen gebunden sind;
die Gruppen R² bis R⁷, welche gleich oder verschieden sind, gewählt sind aus -R⁹ und -L-R⁹, worin R⁹ gewählt ist aus neutralen Gruppen, welche die Wasserlöslichkeit reduzieren, polaren Gruppen, welche die Wasserlöslichkeit erhöhen, funktionellen Gruppen, welche in Markierungsreaktionen verwendet werden können, reaktiven Gruppen, elektronenabgebenden und -abziehenden Gruppen, welche die Absorptions- und Emissionswellenlängen des fluoreszierenden Moleküls verschieben, lipid- und kohlenwasserstoffsolubilisierenden Gruppen, und L gewählt ist aus der Gruppe, bestehend aus einer geraden oder verzweigten C₁₋₂₆-Alkylkette, einem C₂₋₂₀-Monoether oder -Polyether und einer bis zu vier sekundäre Amidbindungen enthaltenden C₂₋₂₀-Atomkette;
R¹ gewählt ist aus Wasserstoff, Aryl, Heteroaryl, Cyano, Nitro, Aldehyd, Halogen, Hydroxy, Alkylgruppen mit 26 Kohlenstoffatomen oder weniger, Amino, quaternäres Amino, Acetal, Ketal, Phosphoryl, Sulfhydryl, wassersolubilisierenden Gruppen und -(CH₂)ₙQ, worin l<n<26 und Q gewählt ist aus Amino, substituiertem Amino, quaternärem Amino, Aldehyd, Acetal, Ketal, Halogen, Cyano, Aryl, Heteroaryl, Hydroxyl, Sulfonat, Sulfat, Carboxylat, Amid, Nitro und Gruppen, welche reaktiv sind mit Amino, Hydroxyl, Aldehyl, Phosphoryl oder Sulfhydrylgruppen;
T eine Verbindungsgruppe ist, so dass 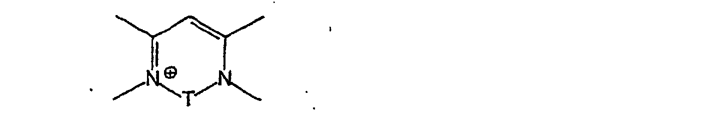 ein 6- oder 7-gliedriger Ring ist;
X und Y gleich oder verschieden sein können und gewählt sind aus zweifach substituiertem Kohlenstoff, Sauerstoff, Schwefel, Selen, CH=CH und N-W, worin N Stickstoff ist und W gewählt ist aus Wasserstoff, einer Gruppe -(CH₂)ₙR⁸, worin n eine ganze Zahl von 1 bis 26 ist und R⁸ gewählt ist aus Wasserstoff, Amino, Aldehyd, Acetal, Ketal, Halogen, Cyano, Aryl, Heteroaryl, Hydroxyl, Sulfonat, Sufat, Carboxylat, substituiertem Amino, quaternärem Amino, Nitro, primärem Amid, substituiertem Amid und Gruppen, welche reaktiv sind mit Amino, Hydroxyl, Carbonyl, Phosphoryl und Sulfhydrylgruppen;
eine der Gruppen Z¹ und Z² eine Bindung bedeutet oder die Atome, welche notwendig sind zur Vervollständigung eines, zweier kondensierter oder dreier kondensierter aromatischer Ringe, wobei jeder Ring fünf oder sechs Atome besitzt, gewählt aus Kohlenstoffatomen, und wahlweise nicht mehr als zwei Sauerstoff-, Stickstoff- und Schwefelatomen, und die andere Gruppe Z¹ oder Z² die Atome bedeutet, welche notwendig sind zur Vervollständigung eines, zweier kondensierter oder dreier kondensierter aromatischer Ringe, wobei jeder Ring fünf oder sechs Atome aufweist, gewählt aus Kohlenstoffatomen und wahlweise nicht mehr als zwei Sauterstoff-, Stickstoff- und Schwefelatomen;
vorausgesetzt, dass mindestens eines von R¹ bis R⁷ eine reaktive Gruppe für die kovalente Reaktion mit einer funktionellen Gruppe auf einem Zielmaterial umfaßt oder eine funktionelle Gruppe für die kovalente Reaktion mit einer reaktiven Gruppe auf einem Zielmaterial umfaßt.

2. Verbindung der Formel: 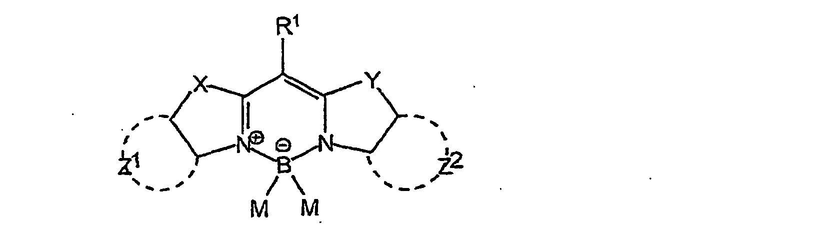 wahlweise substituiert durch Gruppen R⁴ bis R⁷, worin R⁴, R⁵, R⁶ und R⁷ an die X und Y enthaltenden Ringe gebunden sind, oder wahlweise an Atome der Z1-und Z²-Ringstrukturen gebunden sind;
worin M aus F und Cl gewählt ist;
die Gruppen R⁴ bis R⁷, welche gleich oder verschieden sind, gewählt sind aus -R⁹ und -L-R⁹, worin R⁹ gewählt ist aus neutralen Gruppen, welche die Wasserlöslichkeit reduzieren, polaren Gruppen, welche die Wasserlöslichkeit erhöhen, funktionellen Gruppen, welche in Markierungsreaktionen verwendet werden können, reaktiven Gruppen, elektronenabgebenden und -abziehenden Gruppen, welche die Absorptions- und Emissionswellenlängen des fluoreszierenden Moleküls verschieben, lipid- und kohlenwasserstoffsolubilisierenden Gruppen, und L gewählt ist aus der Gruppe, bestehend aus einer geraden oder verzweigten C₁₋₂₆-Alkylkette, einem C₂₋₂₀-Monoether oder -Polyether und einer bis zu vier sekundäre Amidbindungen enthaltenden C₂₋₂₀-Atomkette;
R¹ gewählt ist aus Wasserstoff, Aryl, Heteroaryl, Cyano, Nitro, Aldehyd, Halogen, Hydroxy, Alkylgruppen mit 26 Kohlenstoffatomen oder weniger, Amino, quaternäres Amino, Acetal, Ketal, Phosphoryl, Sulfhydryl, wassersolubilisierenden Gruppen und -(CH₂)ₙQ, worin l<n<26 und Q gewählt ist aus Amino, substituiertem Amino, quaternärem Amino, Aldehyd, Acetal, Ketal, Halogen, Cyano, Aryl, Heteroaryl, Hydroxyl, Sulfonat, Sulfat, Carboxylat, Amid, Nitro und Gruppen, welche reaktiv sind mit Amino, Hydroxyl, Aldehyl, Phosphoryl oder Sulfhydrylgruppen;
X und Y gleich oder verschieden sein können und gewählt sind aus zweifach substituiertem Kohlenstoff, Sauerstoff, Schwefel, Selen, CH=CH und N-W, worin N Stickstoff ist und W gewählt ist aus Wasserstoff, einer Gruppe -(CH₂)ₙR⁸, worin n eine ganze Zahl von 1 bis 26 ist und R⁸ gewählt ist aus Wasserstoff, Amino, Aldehyd, Acetal, Ketal, Halogen, Cyano, Aryl, Heteroaryl, Hydroxyl, Sulfonat, Sufat, Carboxylat, substituiertem Amino, quaternärem Amino, Nitro, primärem Amid, substituiertem Amid und Gruppen, welche reaktiv sind mit Amino, Hydroxyl, Carbonyl, Phosphoryl und Sulfhydrylgruppen;
eine der Gruppen Z¹ und Z² eine Bindung bedeutet oder die Atome, welche notwendig sind zur Vervollständigung eines, zweier kondensierter oder dreier kondensierter aromatischer Ringe, wobei jeder Ring fünf oder sechs Atome besitzt, gewählt aus Kohlenstoffatomen, und wahlweise nicht mehr als zwei Sauerstoff-, Stickstoff- und Schwefelatomen, und die andere Gruppe Z¹ oder Z² die Atome bedeutet, welche notwendig sind zur Vervollständigung eines, zweier kondensierter oder dreier kondensierter aromatischer Ringe, wobei jeder Ring fünf oder sechs Atome aufweist, gewählt aus Kohlenstoffatomen und wahlweise nicht mehr als zwei Sauterstoff-, Stickstoff- und Schwefelatomen;
vorausgesetzt, dass mindestens eines von R¹ und R⁴ bis R⁷ eine reaktive Gruppe für die kovalente Reaktion mit einer funktionellen Gruppe auf einem Zielmaterial umfaßt oder eine funktionelle Gruppe für die kovalente Reaktion mit einer reaktiven Gruppe auf einem Zielmaterial umfaßt.

3. Verbindung der Formel: 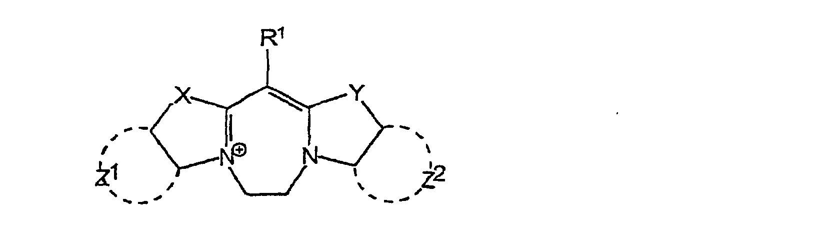 wahlweise substituiert durch Gruppen R² bis R⁷, worin die Gruppen R² bis R³ an die Ethylen-Verbindungsgruppe, 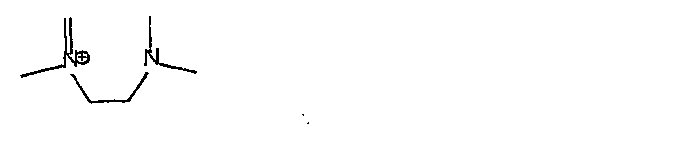 gebunden sind, und die Gruppen R⁴ bis R⁷ and die X und Y enthaltenden Ringe gebunden sind, oder wahlweise an Atome der Z¹- und Z²-Ringstrukturen gebunden sind;
die Gruppen R² bis R⁷, welche gleich oder verschieden sind, gewählt sind aus -R⁹ und -L-R⁹, worin R⁹ gewählt ist aus neutralen Gruppen, welche die Wasserlöslichkeit reduzieren, polaren Gruppen, welche die Wasserlöslichkeit erhöhen, funktionellen Gruppen, welche in Markierungsreaktionen verwendet werden können, reaktiven Gruppen, elektronenabgebenden und -abziehenden Gruppen, welche die Absorptions- und Emissionswellenlängen des fluoreszierenden Moleküls verschieben, lipid- und kohlenwasserstoffsolubilisierenden Gruppen, und L gewählt ist aus der Gruppe, bestehend aus einer geraden oder verzweigten C₁₋₂₆-Alkylkette, einem C₂₋₂₀-Monoether oder -Polyether und einer bis zu vier sekundäre Amidbindungen enthaltenden C₂₋₂₀-Atomkette;
R¹ gewählt ist aus Wasserstoff, Aryl, Heteroaryl, Cyano, Nitro, Aldehyd, Halogen, Hydroxy, Alkylgruppen mit 26 Kohlenstoffatomen oder weniger, Amino, quaternäres Amino, Acetal, Ketal, Phosphoryl, Sulfhydryl, wassersolubilisierenden Gruppen und -(CH₂)ₙQ, worin l<n<26 und Q gewählt ist aus Amino, substituiertem Amino, quaternärem Amino, Aldehyd, Acetal, Ketal, Halogen, Cyano, Aryl, Heteroaryl, Hydroxyl, Sulfonat, Sulfat, Carboxylat, Amid, Nitro und Gruppen, welche reaktiv sind mit Amino, Hydroxyl, Aldehyl, Phosphoryl oder Sulfhydrylgruppen;
X und Y gleich oder verschieden sein können und gewählt sind aus zweifach substituiertem Kohlenstoff, Sauerstoff, Schwefel, Selen, CH=CH und N-W, worin N Stickstoff ist und W gewählt ist aus Wasserstoff, einer Gruppe -(CH₂)ₙR⁸, worin n eine ganze Zahl von 1 bis 26 ist und R⁸ gewählt ist aus Wasserstoff, Amino, Aldehyd, Acetal, Ketal, Halogen, Cyano, Aryl, Heteroaryl, Hydroxyl, Sulfonat, Sufat, Carboxylat, substituiertem Amino, quaternärem Amino, Nitro, primärem Amid, substituiertem Amid und Gruppen, welche reaktiv sind mit Amino, Hydroxyl, Carbonyl, Phosphoryl und Sulfhydrylgruppen;
eine der Gruppen Z¹ und Z² eine Bindung bedeutet oder die Atome, welche notwendig sind zur Vervollständigung eines, zweier kondensierter oder dreier kondensierter aromatischer Ringe, wobei jeder Ring fünf oder sechs Atome besitzt, gewählt aus Kohlenstoffatomen, und wahlweise nicht mehr als zwei Sauerstoff-, Stickstoff- und Schwefelatomen, und die andere Gruppe Z¹ oder Z² die Atome bedeutet, welche notwendig sind zur Vervollständigung eines, zweier kondensierter oder dreier kondensierter aromatischer Ringe, wobei jeder Ring fünf oder sechs Atome aufweist, gewählt aus Kohlenstoffatomen und wahlweise nicht mehr als zwei Sauterstoff-, Stickstoff- und Schwefelatomen;
vorausgesetzt, dass mindestens eines von R¹ bis R⁷ eine reaktive Gruppe für die kovalente Reaktion mit einer funktionellen Gruppe auf einem Zielmaterial umfaßt oder eine funktionelle Gruppe für die kovalente Reaktion mit einer reaktiven Gruppe auf einem Zielmaterial umfaßt.

4. Verbindung nach den Ansprüchen 1, 2 oder 3, worin R⁹ gewählt ist aus:
- neutralen Gruppen, gewählt aus der Gruppe, bestehend aus Wasserstoff und Halogenatomen;
- polaren Gruppen, gewählt aus der Gruppe, bestehend aus Amid, Sulfonat, Sulfat, Phosphat, quaternärem Ammonium, Guanidinium, Hydroxyl, Phosphonat;
- funktionellen Gruppen, gewählt aus der Gruppe, bestehend aus wahlweise substituiertem Amino, Azido, Hydroxyl, Sulfhydryl, Imidazol, Carboxyl und Carbonyl;
- reaktiven Gruppen, gewählt aus der Gruppe, bestehend aus Succinimidylester, Isothiocyanat, Isocyanat, Anhydrid, Halogenacetamid, Maleimid, Sulfonylhalogenid, Phosphoramidit, Säurehalogenid, Acylazid, Alkylimidat, Hydrazid, Arylimidat, Hydroxylaminen, Carbodiimiden;
- elektronenabgebenden und -abziehenden Gruppen, gewählt aus der Gruppe, bestehend aus Amid, Cyano, Nitro, Alkoxy, Sryryl, Aryl und Heteroarylgruppen;
- lipid- und kohlenwasserstoffsolubilisierenden Gruppen, gewählt aus der Gruppe, bestehend aus Alkyl, Aryl und Aralkylgruppen; und
L aus der Gruppe gewählt ist, bestehend aus einer geraden oder verzweigten C₁₋₂₆-Alkyikette, einem C₂₋₂₀-Monoether oder -Polyether und einer bis zu vier sekundäre Amidbindungen enthaltenden C₂₋₂₀-Atomkette.

5. Verbindung nach Anspruch 1, gewählt aus:
i) 6,6'-Disulfo-meso-carboxymethyl-bis-(benzothiazolyl)methinbordifluorid
ii) α-Carboxymethyl-5,5'-disulfo-3,3'-ethylen-oxacyanin
iii) 3,3'-Ethylen-6-sulfothia-5'-carboxymethyl-6'-sulfooxamonomethincyanin
iv) 5-Carboxymethyl-bis-(benzoxazolyl)methinbordifluorid
v) α-Carboxymethyl-3,3'-ethylenthiacyanin
vi) *meso*-Carboxymethyl-benzoxazolyl-benzothiazolylmonomethinbordifluorid

6. Verfahren zur Herstellung einer Verbindung gemäß mindestens einem der Ansprüche 1 bis 5, umfassend das Umsetzen einer Verbindung der Formel (A) oder eine protonierte Form hiervon 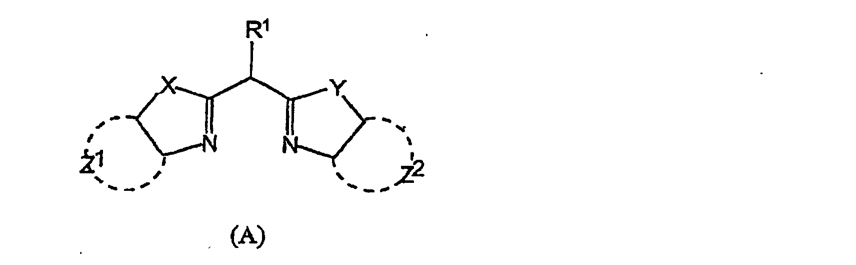 wahlweise substituiert durch Gruppen R⁴ bis R⁷, worin R¹ und R⁴ bis R⁷, X, Y, Z¹ und Z² wie vorangehend definiert sind, mit einer für die Ausbildung der Bindung T geeigneten Verbindung, worin T wie vorangehend definiert ist.

7. Verbindung der Formel 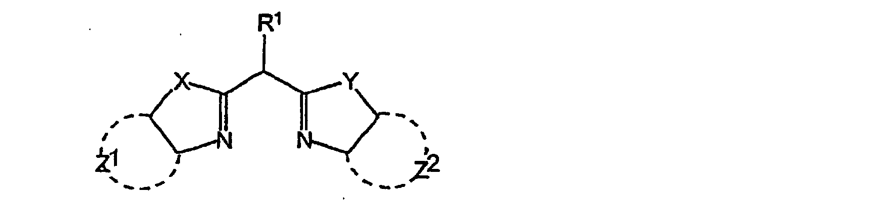 oder eine protonierte Form hiervon;
wahlweise substituiert durch Gruppen R⁴ bis R⁷, worin R⁴, R⁵, R⁶ und R⁷ an die X und Y enthaltenden Ringe gebunden sind, oder wahlweise an Atome der Z¹-und Z²-Ringstrukturen gebunden sind, und worin R¹, R⁴ bis R⁷, X, Y, Z¹ und Z² wie vorangehend definiert sind;
vorausgesetzt, dass mindestens eines von R¹ bis R⁷ eine reaktive Gruppe für die kovalente Reaktion mit einer funktionellen Gruppe auf einem Zielmaterial umfaßt oder eine funktionelle Gruppe für die kovalente Reaktion mit einer reaktiven Gruppe auf einem Zielmaterial umfaßt.

8. Verfahren zum Verleihen von Fluoreszenzeigenschaften an ein nichtpolares Material, wobei das Verfahren die Schritte des Auflösens in dem nichtpolaren Material der Verbindung, wie in irgendeinem der Ansprüche 1-5 genannt, umfaßt, worin mindestens eine der Gruppen R¹ bis R⁷ eine ungeladene Gruppe ist.

9. Verfahren zum Verleihen von Fluoreszenzeigenschaften an ein polares Material, wobei das Verfahren die Schritte des Auflösens in dem polaren Material einer Verbindung, wie in mindestens einem der Ansprüche 1-5 beansprucht, umfaßt, worin mindestens eine der R-Gruppen aus der geladene Gruppen und polare Gruppen umfassenden Gruppe gewählt wird.

10. Verfahren zum Verleihen von Fluoreszenzeigenschaften an ein Zielmaterial, wobei das Verfahren die Schritte des Inkubierens:
i) eines Zielmaterials mit mindestens einer funktionellen Gruppe, gewählt aus der Gruppe, bestehend aus Amino, Hydroxyl, Phosphoryl, Carbonyl und Sulfhydrylgruppen; oder das mindestens eine reaktive Gruppe besitzt, die mit der mindestens einen funktionellen Gruppe kovalent binden kann; und
ii) einer Menge der fluoreszierenden Verbindung, wie in mindestens einem der Ansprüche 1-5 beansprucht, worin mindestens eine der R-Gruppen eine funktionelle Gruppe ist, gewählt aus der Gruppe, bestehend aus Amino, Hydroxyl, Phosphoryl, Carbonyl und Sulfhydryl; oder worin mindestens eine der R-Gruppen eine reaktive Gruppe ist, die mit der mindestens einen funktionellen Gruppe kovalent binden kann;
über einen ausreichenden Zeitraum umfaßt, um es der mindestens einen funktionellen oder reaktiven Gruppe der fluoreszierenden Verbindung zu ermöglichen, kovalent an die mindestens eine reaktive oder funktionelle Gruppe des Zielmaterials zu binden.

11. Verfahren zur Bestimmung der Sequenz einer Nukleinsäure, wobei das Verfahren die Schritte umfaßt:
i) Vorsehen einer Probe der zu sequenzierenden Nukleinsäure, einer Primer-Nukleinsäuresequenz, welche zu mindestens einem Teil der zu sequenzierenden Nukleinsäure komplementär ist, einem Vorrat an Desoxynukleotiden und mindestens einem Didesoxynukleotid zur Terminierung der Sequenzierungsreaktion, und einer Polymerase;
ii) Durchführen von Nukleinsäurekettenverlängerungs- und Kettenterminierungsreaktionen;
iii) Abtrennen der Oligonukleotidfragmente gemäß der Größe;
**dadurch gekennzeichnet, dass** ein oder mehrere der Didesoxynukleotide oder der Primer-Nukleinsäuresequenz mit einer Verbindung gemäß mindestens einem der Ansprüche 1-5 markiert wird.

## Revendications

1. Composé de formule : 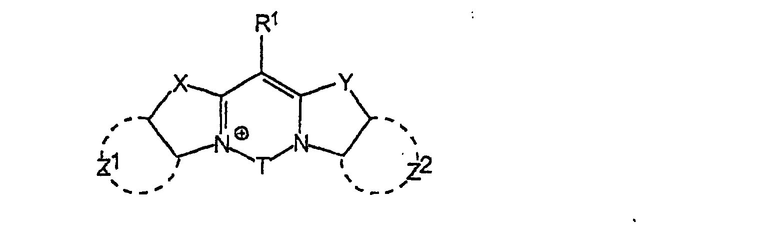 éventuellement substituée par des groupes R⁴ à R⁷, ou par des groupes R² à R⁷ lorsque T contient des atomes de carbone, dans laquelle R² et R³ sont fixés aux atomes de carbone de T et R⁴, R⁵, R⁶ et R⁷ sont fixés aux cycles contenant X et Y ou sont éventuellement fixés aux atomes des structures cycliques Z¹ et Z² ;
les groupes R² à R⁷, qui sont identiques ou différents, sont choisis parmi -R⁹ et -L-R⁹ où R⁹ est choisi parmi des groupes neutres qui réduisent la solubilité dans l'eau, des groupes polaires qui augmentent la solubilité dans l'eau, des groupes fonctionnels que l'on peut utiliser dans des réactions de marquage, des groupes réactifs, des groupes donneurs et attracteurs d'électrons qui déplacent les longueurs d'onde d'absorption et d'émission de la molécule fluorescente, des groupes solubilisateurs dans les lipides et les hydrocarbures, et L est choisi dans le groupe constitué par une chaîne alkyle en C₁₋₂₆ linéaire ou ramifiée, un polyéther ou un monoéther en C₂₋₂₀ et une chaîne atomique en C₂₋₂₀ contenant jusqu'à quatre liaisons amide secondaire ;
R¹ est choisi parmi hydrogène, aryle, héteroaryle, cyano, nitro, aldéhyde, halogéno, hydroxy, alkyle comportant vingt-six atomes de carbone ou moins, amino, amino quaternaire, acétal, cétal, phosphoryle, sulfhydryle, des groupes solubilisateurs dans l'eau et un groupe -(CH₂)ₙQ où l<n<26 et Q est choisi parmi des groupes amino, amino substitué, amino quaternaire, aldéhyde, acétal, cétal, halogéno, cyano, aryle, hétéroaryle, hydroxyle, sulfonate, sulfate, carboxylate, amide, nitro et des groupes réactifs avec des groupes amino, hydroxyle, aldéhyde, phosphoryle ou sulfhydryle;
T représente un groupe de liaison tel que : 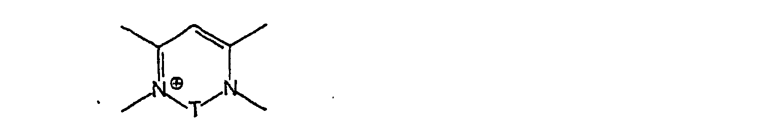 soit un cycle à six ou sept chaînons ;
X et Y peuvent être identiques ou différents et sont choisis parmi des groupes carbone, oxygène, soufre, sélénium, CH=CH disubstitués, et N-W où N représente un atome d'azote et W est choisi parmi un hydrogène, un groupe -(CH₂)ₙR⁸ où n représente un nombre entier de 1 à 26 et R⁸ est choisi parmi hydrogène, amino, aldéhyde, acétal, cétal, halogéno, cyano, aryle, hétéroaryle, hydroxyle, sulfonate, sulfate, carboxylate, amino substitué, amino quaternaire, nitro, amide primaire, amide substitué, et des groupes réactifs avec des groupes amino, hydroxyle, carbonyle, phosphoryle et sulfhydryle ;
un des groupes Z¹ et Z² représente une liaison ou les atomes nécessaires pour compléter un cycle aromatique, deux cycles aromatiques condensés ou trois cycles aromatiques condensés, chaque cycle comportant cinq ou six atomes, choisis parmi des atomes de carbone et, éventuellement, pas plus de deux atomes d'oxygène, d'azote et de soufre et l'autre groupe Z¹ ou Z² représente les atomes nécessaires pour compléter un cycle aromatique, deux cycles aromatiques condensés ou trois cycles aromatiques condensés, chaque cycle comportant cinq ou six atomes, choisis parmi des atomes de carbone et, éventuellement, pas plus de deux atomes d'oxygène, d'azote et de soufre ;
à condition qu'au moins l'un des symboles R¹ à R⁷ comprenne un groupe réactif pour une réaction covalente avec un groupe fonctionnel sur un matériau cible ou comprenne un groupe fonctionnel pour une réaction covalente avec un groupe réactif sur un matériau cible.

2. Composé de formule : 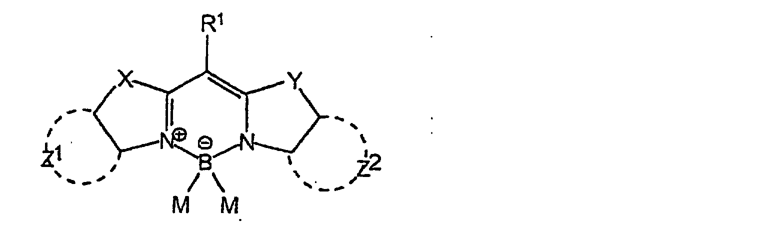 éventuellement substituée par des groupes R⁴ à R⁷, dans laquelle R⁴, R⁵, R⁶ et R⁷ sont fixés aux cycles contenant X et Y ou sont éventuellement fixés aux atomes des structures cycliques Z¹ et Z² , et
dans laquelle M est choisi parmi F et Cl ;
les groupes R⁴ à R⁷, qui sont identiques ou différents, sont choisis parmi -R⁹ et -L-R⁹ où R⁹ est choisi parmi des groupes neutres qui réduisent la solubilité dans l'eau, des groupes polaires qui augmentent la solubilité dans l'eau, des groupes fonctionnels que l'on peut utiliser dans des réactions de marquage, des groupes réactifs, des groupes donneurs et attracteurs d'électrons qui déplacent les longueurs d'onde d'absorption et d'émission de la molécule fluorescente, des groupes solubilisateurs dans les lipides et les hydrocarbures, et L est choisi dans le groupe constitué par une chaîne alkyle en C₁₋₂₆ linéaire ou ramifiée, un polyéther ou un monoéther en C₂₋₂₀ et une chaîne atomique en C₂₋₂₀ contenant jusqu'à quatre liaisons amide secondaire ;
R¹ est choisi parmi hydrogène, aryle, héteroaryle, cyano, nitro, aldéhyde, halogéno, hydroxy, alkyle comportant vingt-six atomes de carbone ou moins, amino, amino quaternaire, acétal, cétal, phosphoryle, sulfhydryle, des groupes solubilisateurs dans l'eau et un groupe -(CH₂)ₙQ où l<n<26 et Q est choisi parmi des groupes amino, amino substitué, amino quaternaire, aldéhyde, acétal, cétal, halogéno, cyano, aryle, hétéroaryle, hydroxyle, sulfonate, sulfate, carboxylate, amide, nitro et des groupes réactifs avec des groupes amino, hydroxyle, aldéhyde, phosphoryle ou sulfhydryle ;
X et Y peuvent être identiques ou différents et sont choisis parmi des groupes carbone, oxygène, soufre, sélénium, CH=CH disubstitués, et N-W où N représente un atome d'azote et W est choisi parmi un hydrogène, un groupe -(CH₂)ₙR⁸ où n représente un nombre entier de 1 à 26 et R⁸ est choisi parmi hydrogène, amino, aldéhyde, acétal, cétal, halogéno, cyano, aryle, hétéroaryle, hydroxyle, sulfonate, sulfate, carboxylate, amino substitué, amino quaternaire, nitro, amide primaire, amide substitué, et des groupes réactifs avec les groupes amino, hydroxyle, carbonyle, phosphoryle et sulfhydryle ;
un des groupes Z¹ et Z² représente une liaison ou les atomes nécessaires pour compléter un cycle aromatique, deux cycles aromatiques condensés ou trois cycles aromatiques condensés, chaque cycle comportant cinq ou six atomes, choisis parmi des atomes de carbone et, éventuellement, pas plus de deux atomes d'oxygène, d'azote et de soufre et l'autre groupe Z¹ ou Z² représente les atomes nécessaires pour compléter un cycle aromatique, deux cycles aromatiques condensés ou trois cycles aromatiques condensés, chaque cycle comportant cinq ou six atomes, choisis parmi des atomes de carbone et, éventuellement, pas plus de deux atomes d'oxygène, d'azote et de soufre ;
à condition qu'au moins l'un des symboles R¹ à R⁷ comprenne un groupe réactif pour une réaction covalente avec un groupe fonctionnel sur un matériau cible ou comprenne un groupe fonctionnel pour une réaction covalente avec un groupe réactif sur un matériau cible.

3. Composé de formule : 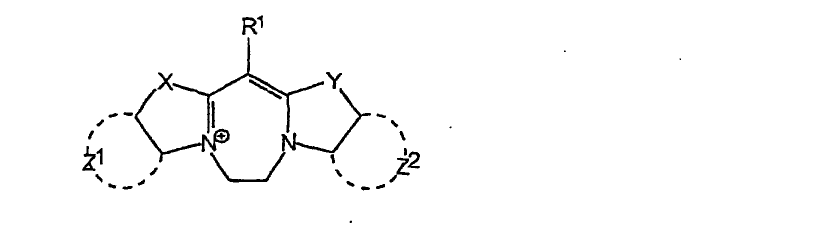 éventuellement substituée par des groupes R² à R⁷, dans laquelle les groupes R² à R³ sont fixés au groupe de liaison éthylène, 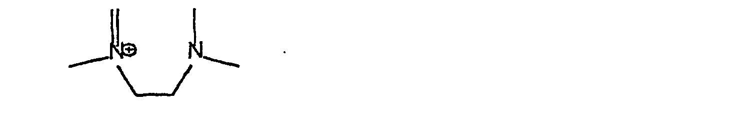 et les groupes R⁴ à R⁷ sont fixés aux cycles contenant X et Y ou sont éventuellement fixés aux atomes des structures cycliques Z¹ et Z² ;
les groupes R² à R⁷, qui sont identiques ou différents, sont choisis parmi -R⁹ et -L-R⁹ où R⁹ est choisi parmi des groupes neutres qui réduisent la solubilité dans l'eau, des groupes polaires qui augmentent la solubilité dans l'eau, des groupes fonctionnels que l'on peut utiliser dans des réactions de marquage, des groupes réactifs, des groupes donneurs et attracteurs d'électrons qui déplacent les longueurs d'onde d'absorption et d'émission de la molécule fluorescente, des groupes solubilisateurs dans les lipides et les hydrocarbures, et L est choisi dans le groupe constitué par une chaîne alkyle en C₁₋₂₆ linéaire ou ramifiée, un polyéther ou un monoéther en C₂₋₂₀ et une chaîne atomique en C₂₋₂₀ contenant jusqu'à quatre liaisons amide secondaire ;
R¹ est choisi parmi hydrogène, aryle, héteroaryle, cyano, nitro, aldéhyde, halogéno, hydroxy, alkyle comportant vingt-six atomes de carbone ou moins, amino, amino quaternaire, acétal, cétal, phosphoryle, sulfhydryle, des groupes solubilisateurs dans l'eau et un groupe -(CH₂)ₙQ où l<n<26 et Q est choisi parmi des groupes amino, amino substitué, amino quaternaire, aldéhyde, acétal, cétal, halogéno, cyano, aryle, hétéroaryle, hydroxyle, sulfonate, sulfate, carboxylate, amide, nitro et des groupes réactifs avec des groupes amino, hydroxyle, aldéhyde, phosphoryle ou sulfhydryle ;
X et Y peuvent être identiques ou différents et sont choisis parmi des groupes carbone, oxygène, soufre, sélénium, CH=CH disubstitués, et N-W où N représente un atome d'azote et W est choisi parmi un hydrogène, un groupe -(CH₂)ₙR⁸ où n représente un nombre entier de 1 à 26 et R⁸ est choisi parmi hydrogène, amino, aldéhyde, acétal, cétal, halogéno, cyano, aryle, hétéroaryle, hydroxyle, sulfonate, sulfate, carboxylate, amino substitué, amino quaternaire, nitro, amide primaire, amide substitué, et des groupes réactifs avec des groupes amino, hydroxyle, carbonyle, phosphoryle, et sulfhydryle ;
un des groupes Z¹ et Z² représente une liaison ou les atomes nécessaires pour compléter un cycle aromatique, deux cycles aromatiques condensés ou trois cycles aromatiques condensés, chaque cycle comportant cinq ou six atomes, choisis parmi des atomes de carbone et, éventuellement, pas plus de deux atomes d'oxygène, d'azote et de soufre et l'autre groupe Z¹ ou Z² représente les atomes nécessaires pour compléter un cycle aromatique, deux cycles aromatiques condensés ou trois cycles aromatiques condensés, chaque cycle comportant cinq ou six atomes, choisis parmi des atomes de carbone et, éventuellement, pas plus de deux atomes d'oxygène, d'azote et de soufre ;
à condition qu'au moins l'un des symboles R¹ à R⁷ comprenne un groupe réactif pour une réaction covalente avec un groupe fonctionnel sur un matériau cible ou comprenne un groupe fonctionnel pour une réaction covalente avec un groupe réactif sur un matériau cible.

4. Composé selon les revendications 1,2 ou 3, dans lequel R⁹ est choisi parmi :
- des groupes neutres choisis dans le groupe constitué par des atomes d'hydrogène et d'halogène ;
- des groupes polaires choisis dans le groupe constitué par des groupes amide, sulfonate, sulfate, phosphate, ammonium quaternaire, guanidinium, hydroxyle, phosphonate ;
- des groupes fonctionnels choisis dans le groupe constitué par des groupes amino, azido, hydroxyle, sulfhydryle, imidazole, carboxyle et carbonyle éventuellement substitués ;
- des groupes réactifs choisis dans le groupe constitué par des groupes ester succinimidylique, isothiocyanate, isocyanate, anhydride, halogénoacétamide, maléimide, halogénure de sulfonyle, phosphoramidite, halogénure d'acide, azide d'acyle, alkylimidate, hydrazide, arylimidate, hydroxylamine, carbodiimide ;
- des groupes donneurs et attracteurs d'électrons choisis dans le groupe constitué par des groupes amide, cyano, nitro, alcoxy, styryle, aryle et hétéroaryle ;
- des groupes solubilisateurs dans les lipides et les hydrocarbures choisis dans le groupe constitué par des groupes alkyle, aryle et aralkyle ; et
L est choisi dans le groupe constitué par une chaîne alkyle en C₁₋₂₆ linéaire ou ramifiée, un polyéther ou un monoéther en C₂₋₂₀ et une chaîne atomique en C₂₋₂₀ contenant jusqu'à quatre liaisons amide secondaire.

5. Composé selon la revendication 1 choisi parmi :
i) le difluorure de 6,6'-disulfo-méso-carboxyméthylbis(benzothiazolyl)méthinebore
ii) l'α-carboxyméthyl-5,5'-disulfo-3,3'-éthylèneoxacyanine
iii) la 3,3'-éthylène-6-sulfothia-5'-carboxyméthyl-6'-sulfooxamonométhinecyanine
iv) le difluorure de 5-carboxyméthylbis(benzoxazolyl)-méthinebore
v) l'α-carboxyméthyl-3,3'-éthylènethiacyanine
vi) le difluorure de méso-carboxyméthylbenzoxazolylbenzothiazolylmonométhinebore.

6. Procédé de production d'un composé selon l'une quelconque des revendications 1 à 5 comprenant l'étape consistant à faire réagir un composé de formule (A), ou une forme protonée de celui-ci, 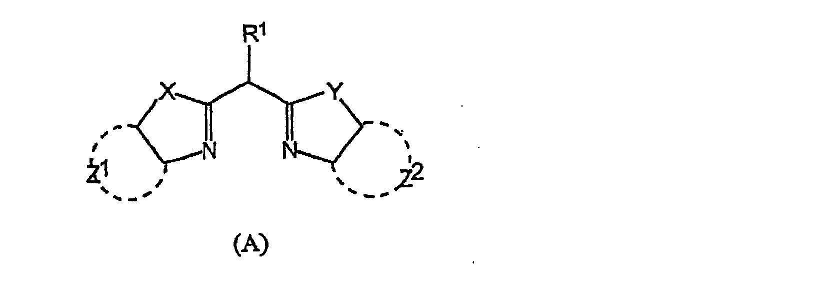 éventuellement substituée par des groupes R⁴ à R⁷, dans laquelle R¹ et R⁴ à R⁷, X, Y, Z¹ et Z² sont tels que définis précédemment, avec un composé approprié pour la formation d'une liaison T, sachant que T est tel que défini précédemment.

7. Composé de formule : 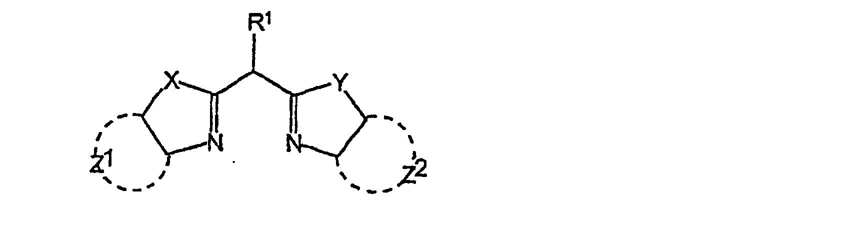 ou forme protonée de celui-ci ;
éventuellement substituée par des groupes R⁴ à R⁷, dans laquelle R⁴, R⁵, R⁶ et R⁷ sont fixés aux cycles contenant X et Y ou sont éventuellement fixés à des atomes des structures cycliques Z¹ et Z² et dans laquelle R¹, R⁴ à R⁷, X, Y, Z¹ et Z² sont tels que définis précédemment ;
à condition qu'au moins l'un des symboles R¹ à R⁷ comprenne un groupe réactif pour une réaction covalente avec un groupe fonctionnel sur un matériau cible ou comprenne un groupe fonctionnel pour une réaction covalente avec un groupe réactif sur un matériau cible.

8. Procédé pour conférer des propriétés fluorescentes à un matériau non polaire, le procédé comprenant les étapes consistant à dissoudre dans le matériau non polaire le composé selon l'une quelconque des revendications 1 à 5, dans lequel au moins un des groupes R¹ à R⁷ est un groupe non chargé.

9. Procédé pour conférer des propriétés fluorescentes à un matériau polaire, le procédé comprenant les étapes consistant à dissoudre dans le matériau polaire un composé selon l'une quelconque des revendications 1 à 5, dans lequel au moins un des groupes R est choisi dans le groupe constitué par des groupes chargés et des groupes polaires.

10. Procédé pour conférer des propriétés fluorescentes à un matériau cible, le procédé comprenant les étapes consistant à faire incuber :
i) un matériau cible ayant au moins un groupe fonctionnel choisi dans le groupe constitué par amino, hydroxyle, phosphoryle, carbonyle et sulfhydryle ; ou ayant au moins un groupe réactif qui peut se lier par covalence avec ledit au moins un groupe fonctionnel, et
ii) une quantité du composé fluorescent selon l'une quelconque des revendications 1 à 5 dans lequel au moins un des groupes R est un groupe fonctionnel choisi dans le groupe constitué par amino, hydroxyle, phosphoryle, carbonyle et sulfhydryle ; ou dans lequel au moins l'un des groupes R est un groupe réactif qui peut se lier par covalence avec ledit au moins un groupe fonctionnel ;
pendant une période de temps suffisante pour permettre audit au moins un groupe fonctionnel ou réactif dudit composé fluorescent de se lier par covalence audit au moins ungroupe réactif ou fonctionnel dudit matériau cible.

11. Procédé pour déterminer la séquence d'un acide nucléique, ledit procédé comprenant les étapes consistant à :
i) fournir un échantillon dudit acide nucléique que l'on veut séquencer, une séquence d'acide nucléique amorce qui est complémentaire d'au moins une partie dudit acide nucléique que l'on veut séquencer, une source de désoxynucléotides et au moins un didésoxynucléotide pour terminer la réaction de séquençage, et une polymérase ;
ii) effectuer les réactions d'élongation de chaîne et de terminaison de chaîne de l'acide nucléique ;
iii) séparer les fragments oligonucléotidiques en fonction de la taille ;
**caractérisé en ce qu'**un ou plusieurs desdits didésoxynucléotides ou ladite séquence d'acide nucléique amorce est marqué par un composé selon l'une quelconque des revendications 1 à 5.
